(19) **Europäisches Patentamt / European Patent Office / Office européen des brevets**

(11) **EP 4 501 339 A1**

(12) # EUROPEAN PATENT APPLICATION

(43) Date of publication:
05.02.2025 Bulletin 2025/06

(21) Application number: 23382797.1

(22) Date of filing: **31.07.2023**

(51) International Patent Classification (IPC):
*A61K 35/741* (2015.01)    *A61K 35/742* (2015.01)
*A61K 35/744* (2015.01)    *A61K 35/745* (2015.01)
*A61K 35/747* (2015.01)    *A61P 17/00* (2006.01)
*A61P 17/02* (2006.01)    *A61P 17/04* (2006.01)
*A61P 17/06* (2006.01)    *A61P 17/08* (2006.01)
*A61P 17/10* (2006.01)    *A23L 33/135* (2016.01)
*A23L 33/14* (2016.01)    *A61K 8/9728* (2017.01)
*A61K 8/99* (2017.01)

(52) Cooperative Patent Classification (CPC):
A23L 33/135; A23L 33/14; A61K 8/99;
A61K 35/741; A61K 35/742; A61K 35/744;
A61K 35/745; A61K 35/747; A61P 17/00;
A61P 17/04; A61P 17/06; A61P 17/08;
A61Q 19/08; C12N 1/20; C12R 2001/01;    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Igen Biolab Group AG**
**6300 Zug (CH)**

(72) Inventors:
• **MOSCOSO DEL PRADO UCELAY, Juan**
**28760 Tres Cantos (ES)**

• **GUARDIA ALBA, María Jesús**
**18198 Huetor Vega (ES)**
• **CARRASCO MARINA, Eva María**
**28100 Alcobendas (ES)**
• **RODRIGUEZ CARBONELL, David**
**28012 Madrid (ES)**
• **RAIGAL VAZQUEZ, Javier**
**28792 Miraflores de la Sierra (ES)**

(74) Representative: **Padial Martinez, Ana Belen**
**Baylos 5.0 Legal Advisors S.L.**
**Av. Diagonal 435, 1.1**
**08008 Barcelona (ES)**

(54) **POSTBIOTIC COMPOSITION FOR SKIN DISORDERS AND COSMETIC USE**

(57) Postbiotic composition for use in the treatment of skin disorders in a subject, comprising microorganisms of the following genera: *Bacillus, Bifidobacterium, Lactobacillus, Saccharomyces* and *Streptococcus,* for topical or oral administration, including a pharmaceutical formulation and cosmetic use.

**EP 4 501 339 A1**

(52) Cooperative Patent Classification (CPC): (Cont.)
C12R 2001/07; C12R 2001/225; C12R 2001/46;
C12R 2001/85

Description

## TECHNICAL FIELD OF THE INVENTION

The present invention describes a postbiotic composition that comprises lysates of probiotic microorganisms for use as a treatment for skin disorders and cosmetic use, which is extremely safe, including during a long period of consumption.

## BACKGROUND

Nowadays, skin disorders are amongst the most common and frequent diseases. These include eczema, psoriasis, acne, rosacea, ichthyosis, vitiligo, urticaria and seborrhoeic dermatitis. The etiology of these diseases as well as their clinical symptoms is so diverse that in many cases they are very difficult to manage.

Due to their very diverse etiology, medical doctors have great difficulty in diagnosing, but above all in managing this type of disease in the most appropriate way possible. In many cases, this type of pathology is treated with corticoids, which are a type of hormone produced by our adrenal glands, the most important of which is cortisol. Corticosteroids are known to have potent anti-inflammatory and immunosuppressive properties, especially when used at pharmacological doses. It is for this reason that they have been widely used to treat various inflammatory diseases, including those affecting the skin.

Nevertheless, it is well known that these treatments have a pernicious effect on the patients. In most cases, there is a high risk of systemic absorption when used topically, with all the risks that this entails. In addition, it can have other side effects when applied inappropriately, such as skin atrophy (thinning), stretch marks and telangiectasias. This calls into question the suitability of these therapies and at the same time serves as an incentive for specialists to seek alternative treatments.

Alternative treatments include the use of natural anti-inflammatories such as turmeric, white willow, devil's claw, ginger, bromelain (pineapple), arnica, omega 3. However, this type of treatment is not as effective in all cases.

This is why, faced with the technical problem of current treatments, there is active work in the field to develop treatments and compositions that can be administered both orally and topically to improve the efficacy of treatments for skin disorders. The following patent document is cited as example:

WO2022185238A1 discloses a topical composition for cosmetic use comprising *Micrococcus luteus* Q24

However, none of the documents found in the prior art disclose the postbiotic composition of the present invention for use in the treatment of skin diseases and as a stable cosmetic use by topical or oral application.

Also noteworthy are Spanish patent applications P201930242 and P201930280 by the same inventors of the current postbiotic composition describing a human intestinal microbiome modulating composition obtained from lysates of probiotic microorganisms and its method of obtaining as well as a food supplement comprising the composition, which has utility in the prevention and treatment of disorders caused, or at least promoted, by intestinal disbiosis, those documents do not disclose or suggest the use of a composition obtained from lysates of probiotic microorganisms for skin disorders

Therefore, the present invention aims to solve the problems of the prior art by using an orally/ or topically administered composition obtained from lysates of probiotic microorganisms for the treatment of skin disorders and cosmetic use in the skin.

## DESCRIPTION OF THE INVENTION

In the present report the expressions "composition of the invention" and "postbiotic composition" are synonymous and are used interchangeably.

In the present report, the term microorganisms include both bacteria and yeast.

Immunomodulatory action is understood, in the context of the present invention, as the set of molecules of the cell walls and lysate of cells and non-viable cells that modulate the innate immune response by the release of cytokines and thereby enhances the immune response against skin disorders.

In the present report the expressions "skin disorders", "skin diseases" and "skin alterations" are synonymous and are used interchangeably.

The terms "treatment" and "treating" as used herein refer to the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, amelioration, stabilization or prevention. The effects of treatment can be measured or assessed as described herein and as known in the art as is suitable for the disease, pathological condition, or disorder involved. Such measurements and assessments can be made in qualitative and/or quantitative terms. Thus, for example, characteristics or features of a disease, pathological condition, or disorder and/or symptoms of a disease, pathological condition, or disorder can be reduced to any effect or to any amount. In the context of a subject suffering from skin disorders, the terms "treatment" and "treating" refer to the medical management of a subject with the intent to cure, ameliorate, or stabilize skin disorders. In the context of a subject at risk of developing skin disorders, the terms "treatment" and "treating" refer to the medical management of a subject with the intent to prevent skin disorders.

In a first aspect, the present invention relates to a postbiotic composition that comprises lysates of probiotic microorganisms in a percentage amount by weight with respect to the total weight of the lysates of microorganisms of the composition:

- 6% to 19% of bacterial lysates of the genus *Bacillus*;
- 4% to 8% of bacterial lysates of the genus *Bifidobacterium*;
- 15% to 25% of bacterial lysates of the genus *Lactobacillus*;
- 50% to 60% of yeast lysates of the genus *Saccharomyces*;
- 1.5% to 7% of bacterial lysates of the genus *Streptococcus.*

for use in the treatment of skin disorders in a subject.

In a particular embodiment, the postbiotic composition of the invention comprises lysates of probiotic microorganisms in a percentage amount by weight with respect to the total weight of the lysates of microorganisms of the composition:

- 13% to 19% of bacterial lysates of the genus *Bacillus*;
- 4% to 8% of bacterial lysates of the genus *Bifidobacterium*;
- 15% to 25% of bacterial lysates of the genus *Lactobacillus*;
- 50% to 60% of yeast lysates of the genus *Saccharomyces;*
- 1.5% to 5% of bacterial lysates of the genus *Streptococcus.*

for use in the treatment of skin disorders.

In accordance with the invention, the composition can comprise between 1% - 99.5% by weight of lysates of microorganisms; particularly 5%-99% by weight of lysates of microorganisms, particularly 10%-96% by weight of lysates of microorganisms, particularly 25%-95% by weight of lysates of microorganisms, or particularly 50%±10% by weight of lysates of microorganisms.

In a particular embodiment, the postbiotic composition of the invention is administered orally or topically.

As indicated previously, the inventors have identified the need to develop a composition that is administered both orally and topically, that is easy to administer and that does not present toxicity to the skin.

Based on this, they have developed a postbiotic composition that can be administered orally or topically, which comprises lysates of microorganisms that can be accompanied by other components and which has proven to be surprisingly beneficial for humans and animals, as it has an immunomodulatory character.

The composition of the invention may comprise additional components or additives or any other substance, such as salts or excipients that facilitate the packaging, preparation and/or administration of the composition but do not affect the capacity of treatment the skin diseases of the postbiotic composition of the invention.

In the context of the present invention, lysates of probiotic microorganisms are understood to be the product obtained after the procedure of culturing and subsequently mechanically or chemically breaking these cells in order to obtain a product with microbial fragments, as well as all the components contained therein. Likewise, this document states that they are lysates of dried probiotic microorganisms, since, thanks to the method of obtaining them, said lysates are subsequently subjected to drying techniques, in such a way that said dried lysates are optionally in the form of a dry powder.

In preferred embodiments of the present invention, the bacterial lysates of the genus *Bacillus* are of the species that are selected from the group consisting of *Bacillus clausii, Bacillus coagulans, Bacillus licheniformis, Bacillus pumilus, Bacillus subtilis, Bacillus mesentericus, Bacillus paralicheniformes,* and combinations thereof. Preferably, *Bacillus coagulans, Bacillus licheniformis, Bacillus mesentericus* and *Bacillus subtilis.*

In other embodiments, the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum,*

*Bifidobacterium infantis, Bifidobacterium animalis,* and combinations thereof, preferably *Bifidobacterium lactis.*

In other embodiments, the bacterial lysates of the genus *Lactobacillus* are of the species that are selected from the group consisting of *Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus, Lactobacillus salivarius, Lactobacillus helvéticus, Lactobacillus gasseri, Lactobacillus johnsonii, Lactobacillus brevis, Lactobacillus kefiri,* and combinations thereof, preferably *Lactobacillus acidophilus, Lactobacillus bulgaricus, Lactobacillus casei, Lactobacillus paracasei,* and *Lactobacillus rhamnosus.*

In other particular embodiments of the present invention, the lysates of yeasts of the genus *Saccharomyces* are of the species that are selected from the group consisting of *Saccharomyces cerevisiae, Saccharomyces boulardi,* and combinations thereof, preferably *Saccharomyces cerevisiae.*

In other particular embodiments of the present invention, the bacterial lysates of the genus *Streptococcus* are of the species *Streptococcus thermophilus.*

In preferred embodiments of the present invention, the composition of the invention comprises bacterial lysates of the genus *Bacillus* in an amount in percentage by weight between approximately 6 % to 19 % of the total lysates of the composition, preferably 13 % to 19 % of the total lysates of the composition. In preferred embodiments, the amount of bacterial lysates of the genus *Bacillus* can be approximately 14%, 15%, 16%, 17%, or 18% of the total lysates of the composition.

In a particular embodiment, the percentage by weight of *Bacillus licheniformis, Bacillus mesentericus* and *Bacillus subtilis* is approximately the same, and is higher than that of *Bacillus coagulans.*

In preferred embodiments of the present invention, the composition of the invention comprises bacterial lysates of the genus *Bifidobacterium* in an amount in percentage by weight between approximately 4% to 8% of the total lysates of the composition, preferably between 5% to 7%. In preferred embodiments, the amount of bacterial lysates of the genus *Bifidobacterium* can be approximately 5%, 6%, 7%, or 8% of the total lysates of the composition.

In preferred embodiments of the present invention, the composition of the invention comprises bacterial lysates of the genus *Lactobacillus* in an amount in percentage by weight between approximately 15% to 25% of the total lysates of the composition, preferably between 20% to 24% of the total lysates of the composition. In preferred embodiments, the amount of bacterial lysates of the genus *Lactobacillus* can be about 20%, 21%, 22%, 23%, 24%, or 25% of the total lysates of the composition.

In another particular embodiment, the percentage by weight of *Lactobacillus rhamnosus* is smaller than the rest of *Lactobacillus* species: *Lactobacillus casei, Lactobacillus paracasei, Lactobacillus bulgaricus* and *Lactobacillus acidophilus*

In a particular embodiment, the percentage by weight of *Lactobacillus casei,* and *Lactobacillus paracasei* is approximately the same, and is higher than that of *Lactobacillus acidophilus* and *Lactobacillus bulgaricus,* which is also approximately the same and is higher than that of *Lactobacillus rhamnosus.*

In preferred embodiments of the present invention, the composition comprises lysates of yeasts of the genus *Saccharomyces* in an amount in percentage by weight of between approximately 50% to 60% of the total lysates of the composition, preferably between 52% to 57% of the total lysates of the composition. In preferred embodiments, the amount of lysates of yeasts of the genus *Saccharomyces* can be about 50%, 51%, 52%, 53%, 54%, 55%, 56%, 57%, 58%, 59%, or 60% of the total lysates of the composition.

In preferred embodiments of the present invention, the composition comprises bacterial lysates of the genus *Streptococcus* in an amount in percentage by weight of between about 1.5% to 5% of the total lysates of the composition, preferably between about 3% to 4% of the total lysates of the composition. In preferred developments, the amount of bacterial lysates of the genus *Streptococcus* can be about 1.5%, 1.6%, 1.7%, 1.8%, 1.9%, 2%, 2.1%, 2.2%, 2.3%, 2.4%, 2.5%, 2.6%, 2.7%, 2.8%, 2.9% or 3% of the total lysates of the composition. In other preferred embodiments, the amount of bacterial lysates of the genus *Streptococcus* can be about 3.5%, 4%, 4.5% or 5% of the total lysates of the composition.

In a particular embodiment of the invention, the composition comprises lysates of probiotic microorganisms in an amount in percentage by weight on the total lysates of the composition:

- approximately 13% to 17% of bacterial lysates of the genus *Bacillus*;
- approximately 4.5% to 7.5% of bacterial lysates of the genus *Bifidobacterium;*
- approximately 20% to 22% of bacterial lysates of the genus *Lactobacillus*;
- approximately 53% to 57% of yeast lysates of the genus *Saccharomyces*;
- approximately 2.5% to 3.5% of bacterial lysates of the genus *Streptococcus.*

This composition is called composition "A".

In another particular embodiment of the invention, the composition comprises lysates of probiotic microorganisms in an amount in percentage by weight on the total lysates of the composition:

- approximately 14% to 17% of bacterial lysates of the genus *Bacillus*;
- approximately 4.6% to 6.5% of bacterial lysates of the genus *Bifidobacterium*;
- approximately 20.5% to 21.8% of bacterial lysates of the genus *Lactobacillus*;
- approximately 55% to 56.5% of yeast lysates of the genus *Saccharomyces*;
- approximately 2.8% to 3.2% of bacterial lysates of the genus *Streptococcus*.

This composition is called composition "B".
In another particular embodiment of the invention, the composition comprises lysates of probiotic microorganisms in an amount in percentage by weight with respect to the total:

- approximately 6% to 19% of bacterial lysates of *Bacillus licheniformis, Bacillus coagulans, Bacillus mesentericus* and *Bacillus subtilis;*
- approximately 4% to 8% of bacterial lysates of *Bifidobacterium lactis;*
- approximately 15% to 25% of bacterial lysates of *Lactobacillus acidophilus, Lactobacillus casei, Lactobacillus bulgaricus, Lactobacillus paracasei,* and *Lactobacillus rhamnosus;*
- approximately 50% to 60% of yeast lysates of *Saccharomyces cerevisiae;*
- approximately 1.5% to 7% of bacterial lysates of *Streptococcus thermophilus.*

This composition is called composition "C".
In another particular embodiment of the invention, the composition comprises lysates of probiotic microorganisms in an amount in percentage by weight with respect to the total:

- approximately 13% to 19% of bacterial lysates of *Bacillus licheniformis, Bacillus coagulans, Bacillus mesentericus* and *Bacillus subtilis;*
- approximately 4% to 8% of bacterial lysates of *Bifidobacterium lactis;*
- approximately 15% to 25% of bacterial lysates of *Lactobacillus acidophilus, Lactobacillus casei, Lactobacillus bulgaricus, Lactobacillus paracasei,* and *Lactobacillus rhamnosus;*
- approximately 50% to 60% of yeast lysates of *Saccharomyces cerevisiae;*
- approximately 1.5% to 5% of bacterial lysates of *Streptococcus thermophilus.*

This composition is called composition "D".
In another particular embodiment of the invention, the composition comprises lysates of probiotic microorganisms in an amount in percentage by weight with respect to the total:

- approximately 13% to 17% of bacterial lysates of *Bacillus licheniformis, Bacillus coagulans, Bacillus mesentericus* and *Bacillus subtilis;*
- approximately 4.5% to 7.5% of bacterial lysates of *Bifidobacterium lactis;*
- approximately 20% to 22% of bacterial lysates of *Lactobacillus acidophilus, Lactobacillus casei, Lactobacillus bulgaricus, Lactobacillus paracasei,* and *Lactobacillus rhamnosus;*
- approximately 53% to 57% of yeast lysates of *Saccharomyces cerevisiae;*
- approximately 2.5% to 3.5% of bacterial lysates of *Streptococcus thermophilus.*

This composition is called composition "E".
In another particular embodiment of the invention, the composition comprises lysates of probiotic microorganisms in an amount in percentage by weight with respect to the total:

- approximately 14% to 17% of bacterial lysates of *Bacillus licheniformis, Bacillus coagulans, Bacillus mesentericus* and *Bacillus subtilis;*
- approximately 4.6% to 6.5% of bacterial lysates of *Bifidobacterium lactis;*
- approximately 20.5% to 21.8% of bacterial lysates of *Lactobacillus acidophilus, Lactobacillus casei, Lactobacillus bulgaricus, Lactobacillus paracasei,* and *Lactobacillus rhamnosus;*
- approximately 55% to 56.5% of yeast lysates of *Saccharomyces cerevisiae;*
- approximately 2.8% to 3.2% of bacterial lysates of *Streptococcus thermophilus.*

This composition is called composition "F".
In another particular embodiment of the invention, the composition comprises lysates of probiotic microorganisms in an amount in percentage by weight with respect to the total:

- approximately 6% to 19% of bacterial lysates of *Bacillus licheniformis, Bacillus coagulans, Bacillus mesentericus* and *Bacillus subtilis,* the percentage by weight of *Bacillus licheniformis, Bacillus mesentericus* and *Bacillus subtilis* is approximately the same, and is higher than that of *Bacillus coagulans.*
- approximately 4% to 8% of bacterial lysates of *Bifidobacterium lactis;*
- approximately 15% to 25% of bacterial lysates of *Lactobacillus acidophilus, Lactobacillus casei, Lactobacillus bulgaricus, Lactobacillus paracasei,* and *Lactobacillus rhamnosus,* the percentage by weight of *Lactobacillus rhamnosus* is smaller than the percentage by weight of *Lactobacillus casei, Lactobacillus paracasei, Lactobacillus bulgaricus* and *Lactobacillus rhamnosus*
- approximately 50% to 60% of yeast lysates of *Saccharomyces cerevisiae;*
- approximately 1.5% to 7% of bacterial lysates of *Streptococcus thermophilus.*

This composition is called composition "G".

In another particular embodiment of the invention, the composition comprises lysates of probiotic microorganisms in an amount in percentage by weight with respect to the total:

- approximately 13% to 19% of bacterial lysates of *Bacillus licheniformis, Bacillus coagulans, Bacillus mesentericus* and *Bacillus subtilis,* the percentage by weight of *Bacillus licheniformis, Bacillus mesentericus* and *Bacillus subtilis* is approximately the same, and is higher than that of *Bacillus coagulans.*
- approximately 4% to 8% of bacterial lysates of *Bifidobacterium lactis;*
- approximately 15% to 25% of bacterial lysates of *Lactobacillus acidophilus, Lactobacillus casei, Lactobacillus bulgaricus, Lactobacillus paracasei,* and *Lactobacillus rhamnosus,* the percentage by weight of *Lactobacillus rhamnosus* is smaller than the percentage by weight of *Lactobacillus casei, Lactobacillus paracasei, Lactobacillus bulgaricus* and *Lactobacillus rhamnosus*
- approximately 50% to 60% of yeast lysates of *Saccharomyces cerevisiae;*
- approximately 1.5% to 5% of bacterial lysates of *Streptococcus thermophilus.*

This composition is called composition "H".

In another particular embodiment of the invention, the composition comprises lysates of probiotic microorganisms in an amount in percentage by weight with respect to the total:

- approximately 13% to 17% of bacterial lysates of *Bacillus licheniformis, Bacillus coagulans, Bacillus mesentericus* and *Bacillus subtilis,* the percentage by weight of *Bacillus licheniformis, Bacillus mesentericus* and *Bacillus subtilis* is approximately the same, and is higher than that of *Bacillus coagulans.*
- approximately 4.5% to 7.5% of bacterial lysates of *Bifidobacterium lactis;*
- approximately 20% to 22% of bacterial lysates of *Lactobacillus acidophilus, Lactobacillus casei, Lactobacillus bulgaricus, Lactobacillus paracasei,* and *Lactobacillus rhamnosus,* the percentage by weight of *Lactobacillus rhamnosus* is smaller than the percentage by weight of *Lactobacillus casei, Lactobacillus paracasei, Lactobacillus bulgaricus* and *Lactobacillus rhamnosus*
- approximately 53% to 57% of yeast lysates of *Saccharomyces cerevisiae;*
- approximately 2.5% to 3.5% of bacterial lysates of *Streptococcus thermophilus.*

This composition is called composition "I".

In another particular embodiment of the invention, the composition comprises lysates of probiotic microorganisms in an amount in percentage by weight with respect to the total:

- approximately 14% to 17% of bacterial lysates of *Bacillus licheniformis, Bacillus coagulans, Bacillus mesentericus* and *Bacillus subtilis,* the percentage by weight of *Bacillus licheniformis, Bacillus mesentericus* and *Bacillus subtilis* is approximately the same, and is higher than that of *Bacillus coagulans.*
- approximately 4.6% to 6.5% of bacterial lysates of *Bifidobacterium lactis;*
- approximately 20.5% to 21.8% of bacterial lysates of *Lactobacillus acidophilus, Lactobacillus casei, Lactobacillus bulgaricus, Lactobacillus paracasei,* and *Lactobacillus rhamnosus,* the percentage by weight of *Lactobacillus rhamnosus* is smaller than the percentage by weight of *Lactobacillus casei, Lactobacillus paracasei, Lactobacillus bulgaricus* and *Lactobacillus rhamnosus*
- approximately 55% to 56.5% of yeast lysates of *Saccharomyces cerevisiae;*
- approximately 2.8% to 3.2% of bacterial lysates of *Streptococcus thermophilus.*

This composition is called composition "J".

In another particular embodiment of the invention, the composition comprises lysates of probiotic microorganisms in an amount in percentage by weight with respect to the total:

- approximately 6% to 19% of bacterial lysates of *Bacillus licheniformis, Bacillus coagulans, Bacillus mesentericus* and *Bacillus subtilis,* the percentage by weight of *Bacillus licheniformis, Bacillus mesentericus* and *Bacillus subtilis* is approximately the same, and is higher than that of *Bacillus coagulans.*
- approximately 4% to 8% of bacterial lysates of *Bifidobacterium lactis;*
- approximately 15% to 25% of bacterial lysates of *Lactobacillus acidophilus, Lactobacillus casei, Lactobacillus bulgaricus, Lactobacillus paracasei,* and *Lactobacillus rhamnosus,* the percentage by weight of *Lactobacillus casei,* and *Lactobacillus paracasei* is approximately the same, and is higher than that of *Lactobacillus acidophilus* and *Lactobacillus bulgaricus,* which is also approximately the same and is higher than that of *Lactobacillus rhamnosus.*
- approximately 50% to 60% of yeast lysates of *Saccharomyces cerevisiae;*
- approximately 1.5% to 7% of bacterial lysates of *Streptococcus thermophilus.*

This composition is called composition "K".

In other particular embodiments of the present invention, additional components or additives may be: carbohydrates (fructose, xylitol, sorbitol, fructooligosaccharides, inulin), antioxidants (resveratrol, beta-carotene), vitamins (Vitamin C, D, K, B1, B9, E, B3, B5, A, B3 , B6, H, D3, B12 biotin, riboflavin, pyridoxine, pantothenic acid), prebiotics (galacto-oligosaccharides, inulin, oligofructose), amino acids (cysteine, tyrosine, resin, methionine, phenylalanine, glycine, glutamine, alanine, carnitine, glutamine, arginine), lipids (eicosapentaenoic acid, docosahexaenoic acid, arachidonic acid), trace elements (sodium, potassium, magnesium, phosphorus, calcium, copper, zinc, manganese, chromium, iodine, selenium), digestive enzymes (papain, amylase, lactase, bromelain), whey.

These micro and macronutrients can modulate the intestinal microbiota and favor the predominance of some species associated with a good state of health, such as species of the genera *Bifidobacterium, Lactobacillus, Akkermansia, Fecalibacterium, Eubacterium, Roseburia, Ruminococcus* and *Blautia.* Among the macronutrients, carbohydrates such as galactooligosaccharides, fructooligosaccharides and inulin have a bifidogenic effect and can modulate microorganisms beneficial to health as well as unsaturated fat, which also increases the beneficial microbiota. Antioxidants such as polyphenols modulate the Firmicutes/Bacteroidetes ratio. Vitamins, such as vitamin A, C, D and E have a positive influence on *Bifidobacteria, Akkermansia* and *Lactobacillus.* Some trace elements such as calcium, magnesium, phosphorus, selenium and zinc have shown an effect on *Akkermansia, Bifidobacterium* and *Ruminococcus.* Prebiotic consumption has been associated with the growth of *Bifidobacterium* and lactic acid bacteria. In general, macro and micronutrients influence the composition and diversity of the gut microbiota.

In a particular embodiment of the invention, the composition of lysates and additional components is that of table 1. Additional components may be one or more of resveratrol, astaxanthin, bromelain, papain, fermented rice starch, corn starch and FOS, each of these components in varying amounts, as shown in Table 1.

Table 1: List of additional components to the composition of the invention per 100 mg of lysates of probiotic microorganisms.

| Composition of lysates of probiotic microorganisms | 100 | Mg |
|---|---|---|
| Resveratrol | Up to 2.55 | Mg |
| Astaxanthin | Up to 25 | Mg |
| Bromelain | Up to 24 | Mg |
| Papain | Up to 35.2 | Mg |
| Fermented rice starch | Up to 110 | Mg |
| Corn starch | Up to 12 | Mg |
| FOS | Up to 70 | Mg |

In the specific example of Table 1, the composition of lysates of probiotic microorganisms would represent 26.4% of the total weight of the composition of the invention.

The postbiotic composition of the invention, being composed of lysates of microorganisms of multiple probiotic species, is intended to treat, this is, to control or at least mitigate skin disorders in a subject, particularly those skin disorders related to inflammatory diseases and/or oxidative stress, more concretely to restore alterations that lead to dysbiosis and have an impact on oxidative and inflammatory processes.

In a particular embodiment, the skin disorders are one or more of the following: eczema, psoriasis, acne, rosacea, ichthyosis, vitiligo, urticaria and seborrhoeic dermatitis, dysbiosis, as well as to control the translocation of microorganisms and/or their components (proteins, nucleic acids). In addition, contrary to compositions comprising probiotics, it avoids the possible colonization by these microorganisms as well as the high cost of keeping them viable during the preparation of the composition of the invention.

As used herein, "subject" includes mammals (e.g., a human, horse, pig, rabbit, dog, sheep, goat, non-human primate, cow, cat, guinea pig or rodent), a fish, a bird or a reptile or an amphibian. The subject can be an invertebrate, more specifically an arthropod (e.g., insects and crustaceans). The term does not denote a particular age or sex. Thus, adult and newborn subjects, as well as fetuses, whether male or female, are intended to be covered. A patient refers to a subject afflicted with a disease or disorder. The term "patient" includes human and veterinary subjects. In one aspect, the compounds described herein can be administered to a subject comprising a human or an animal including, but not limited to, a mouse, dog, cat, horse, bovine or ovine and the like, that is in need of alleviation or amelioration from a recognized medical condition.

In particular embodiments of the present invention, the composition of the invention, is obtained by a method comprising the following steps:

- Cultivate the microbial species selected for the preparation of the composition using the standard conditions established for the microbial species listed in this document;
- Filter or centrifuge the microbial species until a suitable biomass of the selected microorganisms is obtained;
- Lyse through freezing and thawing and sonication cycles of the biomasses obtained. until securing at least 90% or 91% or 92% or 94% of bacterial lysates, preferably at least 95%, or 96% or 97% or 98% and more preferably, 99% or 100%;
- Mix the different strains in the proportions selected according to the percentage by weight to obtain the composition of lysates of probiotic microorganisms of the invention.

A skilled person would know how to find the appropriate protocol for the growth of the microorganisms of the composition, as well as the protocol for their lysis, since these are common procedures in the prior art.

In a particular embodiment, the method of obtaining the composition of the invention comprises an additional step: drying by atomization or lyophilization the biomasses of bacterial lysates obtaining a product in powder form comprising all the bacterial strains selected to prepare the composition object of the invention.

In a particular embodiment, the composition of the invention is presented in the form of dry powder.

When the composition of the invention is presented in the form of dry powder, it is necessary to dissolve it in water or any other liquid that does not affect the properties of the components of the composition prior to its intake by the patient. The advantages of dry powder are that it is easier to transport and to store, and it also increases the stability and durability of the composition. In the present document, dry powder means that the humidity level is less than 10%, preferably less than 5%, even more preferably less than 1%.

In particular embodiments, the composition of the invention is obtained from cultures of probiotic microorganisms of the genera described in the present document, which comprise a certain amount of Colony Forming Units (CFU). In the context of the present invention, a Colony Forming Unit is a term of microbiology. It is an indicator of the amount of live microorganisms present in a medium.

The process of obtaining lysates comprises a thermal treatment, alternating heating and cooling cycles. Each batch of viable cell culture is resuspended in distilled water in a 1:2 ratio and subjected to a sterilization cycle in an autoclave at 121°C for 20 minutes. They are then frozen at -20°C for 12 hours and thawed at room temperature the next day.

Once thawed, they undergo a cell rupture treatment by sonication for 20min, at an output power of 500 W at an amplitude of 40% and 10 seconds of pause, obtaining a mass of lysed cells from the probiotic batch (Qsonica, Q500). The final solution is freeze-dried and ground to obtain a lysate of the powdery probiotic microorganism. The powder is kept in a cool environment away from heat. The degree of survival in no case exceeds a final viability of approximately 7 E3 CFU/ml, which implies practically 100% cell death.

In particular embodiments of the present invention, one starts with a quantity of bacteria of the genus *Bacillus* comprising approximately between 3.49% to 20.94% of CFU with respect to the total CFU of the composition, preferably between approximately 9.98% and 15.75%. In absolute values, the culture grown, before proceeding to the lysis of the cells, presents between 1E+11 and 5E+11 cells, preferably between 2E+11 and 3E+11, most preferably approximately 2.65E+11.

In another particular embodiment, when the species of the genus *Bacillus* are: *Bacillus coagulans, Bacillus licheniformis, Bacillus mesentericus* and *Bacillus subtilis,* each of the grown cultures presents, before proceeding to the lysis of the cells, between 1E+10 and 7E+10 of *Bacillus coagulans* cells, between 6E+10 and 3E+11 of *Bacillus licheniformis* cells, between 4E+10 and 8E+10 of *Bacillus mesentericus* cells and between 4E+10 and 1E+11 of *Bacillus subtilis* cells.

In particular embodiments of the present invention, one starts with a number of bacteria of the genus *Lactobacillus* comprising between 28.64% to 66.82% of CFU with respect to the total CFU of the composition, preferably between 40.66% to 57.36%. In absolute values, the culture grown, before proceeding to the lysis of the cells, presents between 8E+11 and 3E+12 cells, preferably between 9E+11 and 2E+12, most preferably approximately 1.07E+12.

In another particular embodiment, when the species of the genus *Lactobacillus* are: *Lactobacillus acidophilus, Lactobacillus bulgaricus, Lactobacillus casei, Lactobacillus fermentum, Lactobacillus reuteri* and *Lactobacillus rhamnosus,* each of the grown cultures presents, before proceeding to the lysis of the cells, between 5E+10 and 9E+10 cells of *Lactobacillus acidophilus,* between 5E+10 and 9E+10 cells of *Lactobacillus bulgaricus,* between 2E+11 and 8E+11 cells of *Lactobacillus casei,* between 3E+10 and 9E+10 cells of *Lactobacillus fermentum,* between 3E+10 and 9E+10 cells of *Lactobacillus reuteri* and between 1E+11 and 6E+11 cells of *Lactobacillus rhamnosus.*

In particular embodiments of the present invention, one starts with a number of bacteria of the genus *Streptococcus* comprising between 5.72% to 38.15% of CFU with respect to the total CFU of the composition, preferably between 10.98% to 20.75%. In absolute values, the culture grown, before proceeding to the lysis of the cells, presents between 1E+11 and 6E+11 cells, preferably between 2E+11 and 4E+11, most preferably approximately 3.00E+11. In a particular embodiment, the species of *Streptococcus* is *Streptococcus thermophilus.*

In particular embodiments of the present invention, one starts with a quantity of bacteria of the genus *Saccharomyces* comprising between 11.44% to 24.79% of CFU with respect to the total culture, preferably between 19.04% to 21.75%. In absolute values, the culture grown, before proceeding to the lysis of the cells, presents between 1E+11 and 7E+11 cells, preferably between 4E+11 and 6E+11, most preferably approximately 5,20E+11. In a particular embodiment, the species of *Saccharomyces* is *Saccharomyces cerevisiae.*

In particular embodiments of the present invention, one starts with a quantity of bacteria of the genus *Bifidobacterium* comprising between 10.20% to 53.64% of CFU with respect to the total culture, preferably between 15.99% to 18.75%. In absolute values, the culture grown, before proceeding to the lysis of the cells, presents between 1E+11 and 7E+11 cells, preferably between 4E+11 and 6E+11, most preferably approximately 4.50E+11. In a particular embodiment, the species of *Bifidobacterium* is *Bifidobacterium lactis.*

These probiotic bacteria are cultured under standard conditions, as set out in the culture protocols published by the Spanish Collection of Type Cultures (CECT), indicated for each of the bacterial species described in this document.

Once these microorganisms are cultured, they are subjected to a lysis procedure. First of all, the microbial cells undergo heat treatment. Each batch of viable cell culture undergoes a sterilization cycle in an autoclave at 121°C for 20 to 30 minutes. This temperature denatures and coagulates the proteins by inactivating them. In addition, it causes damage to membranes, aggregation of ribosomes, breaking of DNA strands and inactivation of enzymes. Once cold, they undergo a cell rupture treatment by sonication for a period of 15 to 20min, at an output power of between 450 to 550 W, at an amplitude of between 38 to 43% and a period of 8 to 15 seconds of pause, obtaining a mass of lysed cells from the probiotic batch (Qsonica, Q500). This achieves the breakage of intermolecular interactions and DNA fragmentation. Optionally, the final solution is freeze-dried and ground to obtain a lysate of the powdery probiotic microorganism. The powder is kept in a cool environment away from heat.

The composition, in accordance with the invention, is the result of the combination of probiotic microorganisms that, after a process of growth and lysis, generates an extract consisting of a set of metabolites, proteins, DNA fragments and other components, such as, for example, peptidoglycans, which through efficient dosing is able to alter and modify in a surprising way the microbiota of the host through several mechanisms in such a way that it activates the immune system, reversing dysbiosis and surprisingly helping to improve skin disorders.

The technical effect deriving from the composition of the invention is that it acts directly on the microbiota of a subject. Thanks to this restorative and modulating action, the inventors have confirmed that, surprisingly, intervening on the microbiota of a subject can significantly improve the health state of said subject, and in particular the condition of skin disorders, as demonstrated by the experimental results described in the present application.

In a particular embodiment, when the composition of the invention is in powder form, the composition, in line with the first aspect of the invention, can be presented in sealed sachets, which is in itself conventional in the food and pharmaceutical industry.

In the context of the present invention, a dose is defined as the amount of the composition of the invention that is to be effective, efficacious and safe for the subject and to solve the health problem for which it has been indicated.

Thus, in the context of the present invention, it is established that at least one dose must be administered, preferably at least one daily dose, between 100mg to 80000mg of the composition of the invention, including any of the compositions A-K. In preferred embodiments, a dosage may comprise between 100mg to 50000mg of the composition of the invention, including any of the compositions A-K; in another preferred embodiment, a dosage may comprise between 100mg to 2000mg of the composition of the invention, including any of the compositions A-K; in another preferred embodiment, a dosage may comprise between 100mg to 700mg of the composition of the invention, including any of the compositions A-K. In particular embodiments of the present invention, a dosage may comprise approximately 120mg, 130mg, 140mg, 150mg, 160mg, 170mg, 180mg, 190mg, 200mg 210mg, 220mg, 230mg, 240mg, 250mg, 260mg, 270mg, 280mg, 290mg, 300mg 310mg, 320mg, 330mg, 340mg, 350mg, 360mg, 370mg, 380mg, 390mg, 400mg, 410mg, 420mg, 430mg, 440mg, 450mg, 460mg, 470mg, 480mg, 490mg, 500mg, 510mg, 520mg, 530mg, 540mg, 550mg, 560mg, 570mg, 580mg, 590mg, 600mg, 610mg, 620mg, 630mg, 640mg, 650mg, 660mg, 670mg, 680mg, or 690mg of the composition of the invention, including any of the compositions A-K.

In a particular embodiment, you can administer at least one dose of the composition of the invention, including any of the compositions A-K, daily, or every other day, or every 3 or 4 days, following any of the concentrations of the preceding paragraph.

In a particular embodiment the dosage is at least 100 mg/day, or at least 100 md/day, or between 80 and 150 mg/day, or between 80 and 200 mg/day.

In another additional embodiment, the administration of the postbiotic composition of the invention, including any of the compositions A-K, is daily for at least 2 weeks, preferably daily for at least 3 weeks, even more preferably daily for at least 4 weeks, even more preferably daily for at least 5 weeks, even more preferably daily for at least 6 weeks, even more preferably daily for at least 7 weeks, even more preferably daily for at least 8 weeks, or daily for at least 9 weeks, or daily for at least 10 weeks, or daily for at least 11 weeks, or daily for at least 12 weeks.

Preferably the composition of the invention, including any of the compositions A-K, is administered in an amount between 100mg to 700mg per dose and between 1 to 6 doses per day for at least 2 weeks.

In another additional embodiment, the treatment with the postbiotic composition is carried out before, simultaneously or after the treatment of the patient with any other skin treatment. When the treatment with the composition of the invention manages to improve the general condition of the patient, any other skin treatment can be performed if the doctors consider that the patient can withstand them.

An additional object of the invention relates to a pharmaceutical formulation comprising an effective pharmaceutical amount of the composition, in accordance with the first aspect of the invention, and at least one pharmaceutically acceptable excipient for use in the treatment of skin disorders.

In the context of the present invention, the expression "pharmaceutical composition" refers to a formulation that has been adapted to deliver a predetermined dose of one or more useful therapeutic agents to a cell, a group of cells, an organ, or a tissue.

The expression "effective pharmaceutical quantity", as used herein, is understood as a nontoxic amount, capable of providing a therapeutic effect. The exact amount required will vary from one subject to another, depending on the species, age and general condition of the subject, the severity of the disease being treated, the particular compound used, its mode of administration, and the like. Therefore, it is not possible to specify an exact "effective quantity". However, an appropriate effective amount can be determined by a skilled person through routine experimentation. In the context of compounds and compositions for the prevention of infection, the pharmaceutically effective quantity can refer to the amount necessary to relieve the symptoms of cancer suffered by the subject and improve their quality of life.

Also, in the context of the present invention, "pharmaceutically acceptable excipient" means a therapeutically inactive substance that is said to be used to incorporate the active ingredient, and that is acceptable to the patient from a pharmacological/toxicological point of view and to the pharmaceutical chemist who manufactures it from a physical/-chemical point of view, with respect to composition, formulation, stability, patient acceptance and bioavailability.

The pharmaceutical formulation of the invention can be suited for topical Administration or oral administration. This is, the at least one pharmaceutical acceptable excipient is suitable for topical or oral administration.

A topical administration is that that is applied directly over the skin disorder As it will be shown later, the composition of the invention is effective as a direct application (Examples 1 and 3) and as a oral administration (Example 2) such as s food supplement. Formulations for topical administration can include ointments, lotions, creams, gels, drops, suppositories, sprays, serums, oils liquids, and powders. Conventional pharmaceutical carriers, aqueous, powder or oily bases, thickeners and the like can be necessary or desirable.

In a particular embodiment, the formulation is administered as a cream. It may contain anti-inflammatories such as aloe vera, skin regenerators such as vitamin D, antioxidants such as vitamin C, moisturisers such as glycerine, collagen stimulators such as glycolic acid, and anti-ageing ingredients such as peptides, retinol and hyaluronic acid. For example, the ingredients that can be used are: water, Glycerin, Butyrospermun Parkii Butter, Cetyl Palmitate, Dimethicone, Sodium, Polyacrylate, Phenoxyethanol, linalool, Ctitronellol, alpha-isomethyl Ionone, geraniol, mentol, parfum, Vitamin D, Aloe Vera, Vitamin E, Almond oil.

Formulations for oral administration can include powders or granules, suspensions or solutions in water or non-aqueous media, capsules, sachets, or tablets. Thickeners, flavorings, diluents, emulsifiers, dispersing aids or binders can be desirable.

Another additional object of the invention relates to a food supplement comprising the composition of the invention for use in the treatment of a patient with a skin disorder, including any of the compositions "A-K".

In the context of the present invention, what is understood by food supplement is a food product whose purpose it is to supplement the normal diet, and which consists of concentrated sources of nutrients or other substances, that has a nutritional or physiological effect, in simple or combined form, and is marketed in dosed form, ie capsules, tablets, pills and other similar means, sachets of powders, ampoules of liquid, dropper bottles, and other similar forms of liquids and powders to be taken in small unit quantities, as defined in Directive 2002/46/EC of the European Parliament.

In a particular embodiment, the food supplement, in accordance with the invention, is in capsule form, such as conventional gelatin capsules, inside which the composition in powder form is contained.

In particular embodiments where the composition is presented in sealed sachets, said composition can be administered to the patient dissolved or suspended in a liquid, preferably in an aqueous liquid, and more preferably, in beverages such as fruit juices, milk, water. In addition, it can also be mixed with foods such as yoghurt, liquid yoghurt, soups, purees, creams, or porridges. These foods must be at optimum temperature to be consumed, and never heated after having added the composition of the invention.

In a third aspect, the invention relates to the composition defined herein for cosmetic use in skin conditions by improving the appearance of skin or at least one sign of aging.

The compositions described herein are useful for improving the appearance of skin or improving signs of aging of the skin. Improving the appearance of skin or a sign of aging may include effects that include, but are not limited to, that skin looks more radiant, skin looks healthier, skin feels more hydrated, pores are reduced in size, skin feels softer, skin looks clearer, moisture is increased, sebum production is decreased, and reducing the appearance of wrinkles, dryness, roughness, dullness, spots including age spots, and impurities.

In a particular embodiment the postbiotic composition of the invention, or the pharmaceutical composition comprising thereof is useful in the treatment and/or prevention of dehydrated skin; skin with redness; aged or photo-aged skin; skin ageing, in particular photo-ageing; and disorders linked to radical attacks linked to chemical or atmospheric pollution.

Pore size, skin moisture content, sebum productions, wrinkles, spots, and impurities may all be measured using a Skin analyser device (eg. (Dermo Prime (dp)/viso, CHOWIS, South Korea). An AI powered skin analyser device quantitatively measures changes in various skin parameters such as hydration, sebum, pores, wrinkles, spots/pigmentation, impurities, keratin, skin tone, blackheads, and skin sensitivity. The device is equipped with a moisture sensor and utilizes advanced optic technology with interchangeable lenses to measure up to 10 different skin measurements and performs accurate analysis of high resolution images via the DermoBella app installed in android or OS tablets or smartphones. The device is used according to the protocols as set out in the Examples herein. Skin parameter measurements are taken at the same skin site each time.

The degree of impurities such as redness can be analyzed by measuring the amount of porphyrin. It is indicated as scarlet, orange light in response to a specific wavelength range of UV light.

Impurities are seen as either a scarlet colour or a yellow-green colour. These can be analyzed separately, but the device combines the two and classifies them as impurities. The index is computed by a percentage against image size. There are no arbitrary values that classify the degree of redness severity based on the amount of porphyrin detected.

Therefore, the main advantages deriving from the postbiotic composition of the invention are the following:

- the physiological functions of the microbiota of humans and animals are supplemented and/or complemented;

- the alterations that lead to dysbiosis and have an impact on oxidative and inflammatory processes are restored;

- the fact that the product is administered topically, makes the present composition, and its surprising effect, a very advantageous alternative to current skin treatments;

- the present composition comprises lysates of microorganisms, i.e. it does not contain living organisms, which makes the present composition a safe alternative, because it avoids the possible dangers of colonising organisms, and it does not have the toxicity that a conventional drug can have.

Additionally, the invention comprises the following clauses:

1. A postbiotic composition that comprises lysates of probiotic microorganisms in a percentage amount by weight with respect to the total weight of the lysates of microorganisms of the composition:

- 6% to 19% of bacterial lysates of the genus *Bacillus*;
- 4% to 8% of bacterial lysates of the genus *Bifidobacterium*;
- 15% to 25% of bacterial lysates of the genus *Lactobacillus*;
- 50% to 60% of yeast lysates of the genus *Saccharomyces*;
- 1.5% to 7% of bacterial lysates of the genus *Streptococcus*.

for use in the treatment of skin disorders.

2. The composition for use, according to the preceding clause, comprising lysates of probiotic microorganisms in an amount in percentage by weight with respect to the total weight of the lysates of microorganisms of the composition:

- 13% to 19% of bacterial lysates of the genus *Bacillus*;
- 4% to 8% of bacterial lysates of the genus *Bifidobacterium*;
- 15% to 25% of bacterial lysates of the genus *Lactobacillus*;
- 50% to 60% of yeast lysates of the genus *Saccharomyces*;
- 1.5% to 5% of bacterial lysates of the genus *Streptococcus*.

3. The composition for use, according to any of the preceding clauses, that is administered orally or topically.

4. The composition for use, according to any of the preceding clauses, that comprises between 1% and 99.5% by weight of lysates of microorganisms, particularly 5%-99% by weight of lysates of microorganisms, particularly 10%-96% by weight of lysates of microorganisms, 25%-95% by weight of lysates of microorganisms, or particularly 50%+10% by weight of lysates of microorganisms.

5. The composition for use, according to any of the preceding clauses, wherein the bacterial lysates of the genus *Bacillus* are of the species that are selected from the group consisting of *Bacillus clausii, Bacillus coagulans, Bacillus licheniformis, Bacillus pumilus,* Bacillus *subtilis, Bacillus mesentericus, Bacillus paralicheniformes,* and combinations thereof.

6. The composition for use, according to any of the preceding clauses, wherein the bacterial lysates of the genus *Bacillus* are of the *Bacillus coagulans, Bacillus licheniformis, Bacillus mesentericus* and *Bacillus subtilis*

7. The composition for use, according to any of the preceding clauses, wherein the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof.

8. The composition for use, according to any of the preceding clauses, wherein the bacterial lysates of the genus *Bifidobacterium* are of the species *Bifidobacterium lactis.*

9. The composition for use, according to any of the preceding clauses, wherein the bacterial lysates of the genus *Lactobacillus* are of the species that are selected from the group consisting of *Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus Lactobacillus salivarius, Lactobacillus helvéticus, Lactobacillus gasseri, Lactobacillus johnsonii, Lactobacillus brevis, Lactobacillus kefiri,* and combinations thereof.

10. The composition for use, according to any of the preceding clauses, wherein the bacterial lysates of the genus *Lactobacillus* are of the species *Lactobacillus acidophilus, Lactobacillus bulgaricus, Lactobacillus casei, Lactobacillus paracasei,* and *Lactobacillus rhamnosus.*

11. The composition for use, according to any of the preceding clauses, wherein the lysates of yeast genus *Saccharomyces* are of the species that are selected from the group consisting of *Saccharomyces cerevisiae, Saccharomyces boulardi,* and combinations thereof.

12. The composition for use, according to any of the preceding clauses, wherein the lysates of yeast genus *Saccharomyces* are of the species *Saccharomyces cerevisiae.*

13. The composition for use, according to any of the preceding clauses, wherein the bacterial lysates of the genus *Streptococcus* are of the species *Streptococcus thermophilus, Streptococcus salivarius,* preferably *Streptococcus thermophilus.*

14. The composition for use, according to any of the preceding clauses, wherein the bacterial lysates of the genus *Bacillus* are approximately 13% to 19% of the total lysates of the composition.

15. The composition for use, according to any of the preceding clauses, wherein the percentage by weight of *Bacillus licheniformis, Bacillus mesentericus* and *Bacillus subtilis* is approximately the same, and is higher than that of *Bacillus*

*coagulans.*

16. The composition for use, according to any of the preceding clauses, wherein bacterial lysates of the genus *Bifidobacterium* are approximately 5% to 7% of the total lysates of the composition.

17. The composition for use, according to any of the preceding clauses, wherein bacterial lysates of the genus *Lactobacillus* are about 20% to 24% of the total lysates of the composition.

18. The composition for use, according to any of the preceding clauses, wherein the percentage by weight of *Lactobacillus rhamnosus* is smaller than the percentage by weight of *Lactobacillus casei, Lactobacillus paracasei, Lactobacillus bulgaricus* and *Lactobacillus acidophilus.*

19. The composition for use, according to any of the preceding clauses, wherein the percentage by weight of *Lactobacillus casei,* and *Lactobacillus paracasei* is approximately the same, and is higher than that of *Lactobacillus acidophilus* and *Lactobacillus bulgaricus,* which is also approximately the same and is higher than that of *Lactobacillus rhamnosus.*

20. The composition for use, according to any of the preceding clauses, wherein the lysates of yeast of the genus *Saccharomyces* are approximately between 52% to 57% of the total lysates of the composition.

21. The composition for use, according to any of the preceding clauses, wherein the lysates of yeast of the genus *Streptococcus* are approximately between 3% to 4% of the total lysates of the composition.

22. The composition for use, according to any of the preceding clauses, wherein the composition comprises lysates of probiotic microorganisms in an amount in percentage by weight of the total lysates of the composition according to composition A.

23. The composition for use, according to any of the preceding clauses, wherein the composition comprises lysates of probiotic microorganisms in an amount in percentage by weight of the total lysates of the composition according to composition B.

24. The composition for use, according to any of the preceding clauses 1 to 21, wherein the composition comprises lysates of probiotic microorganisms in an amount in percentage by weight of the total lysates of the composition according to composition C.

25. The composition for use, according to any of the preceding clauses 1 to 21 and 24, wherein the composition comprises lysates of probiotic microorganisms in an amount in percentage by weight of the total lysates of the composition according to composition D.

26. The composition for use, according to any of the preceding clauses 1 to 21 and 24 to 25, wherein the composition comprises lysates of probiotic microorganisms in an amount in percentage by weight of the total lysates of the composition according to composition E.

27. The composition for use, according to any of the preceding clauses, wherein the composition comprises lysates of probiotic microorganisms in an amount in percentage by weight of the total lysates of the composition according to composition F.

28. The composition for use, according to any of the preceding clauses 1 to 21 and 24 wherein the composition comprises lysates of probiotic microorganisms in an amount in percentage by weight of the total lysates of the composition according to composition G.

29. The composition for use, according to any of the preceding clauses 1 to 21 and 24 to 25, and 28, wherein the composition comprises lysates of probiotic microorganisms in an amount in percentage by weight of the total lysates of the composition according to composition H.

30. The composition for use, according to any of the preceding clauses 1 to 22 and 24 to 26 and 28 to 29, wherein the composition comprises lysates of probiotic microorganisms in an amount in percentage by weight of the total lysates of the composition according to composition I.

31. The composition for use, according to any of the preceding clauses, wherein the composition comprises lysates of probiotic microorganisms in an amount in percentage by weight of the total lysates of the composition according to composition J.

32. The composition for use, according to any of the preceding clauses 1 to 21 and 24 and 28, wherein the composition comprises lysates of probiotic microorganisms in an amount in percentage by weight of the total lysates of the composition according to composition K.

33. The composition for use, according to any of the preceding clauses, wherein the skin disorders are oxidative stress and/or inflammatory diseases of the skin.

34. The composition for use, according to any of the preceding clauses, wherein the skin disorders are one or more of the following: eczema, psoriasis, acne, rosacea, ichthyosis, vitiligo, urticaria and seborrhoeic dermatitis, and dysbiosis.

35. A procedure for obtaining a postbiotic composition for use in the treatment of skin disorders, according to any of the clauses 1 to 34 comprising:

- Cultivating the selected microbial species

- Filter or centrifuge the cultures until an adequate biomass of the selected microorganisms is obtained

- Lysis through cycles of freezing and thawing, and sonication of the obtained the biomasses

36. The process, according to the preceding clause, comprising drying by atomization or lyophilization of the biomasses of bacterial lysates obtaining a product in the form of dry powder.

37. A pharmaceutical formulation comprising an effective pharmaceutical quantity of the postbiotic composition, according to any of the preceding clauses 1 to 34, and at least one pharmaceutically acceptable excipient for use in the treatment of skin disorders.

38. The pharmaceutical formulation for use, according to the preceding clause, wherein the at least one pharmaceutical acceptable excipient is suitable for topical or oral administration.

39. The pharmaceutical formulation for use, according to any of the preceding clauses 37 to 38, wherein formulations for topical administration are ointments, lotions, creams, gels, drops, suppositories, sprays, serums, oils liquids, and powders.

40. The pharmaceutical formulation for use, according to any of the preceding clauses 37 to 39, wherein the formulation is administered as a cream and comprises anti-inflammatories, skin regenerators, antioxidants, moisturisers, collagen stimulators, and anti-ageing ingredients.

41. The pharmaceutical formulation for use, according to any of the preceding clauses 37 to 38, wherein formulations for oral administration are powders or granules, suspensions or solutions in water or non-aqueous media, capsules, sachets, or tablets

42. A food supplement comprising the postbiotic composition, according to any of the clauses 1 to 34.

43. Cosmetic use of the postbiotic composition according to any of the preceding clauses 1 to 34, or the pharmaceutical formulation according to any of the preceding clauses 37 to 41 by improving the appearance of skin or at least one sign of skin aging

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1 , about 2 and about 3"). When a listing of values is described the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in examples below

## BRIEF DESCRIPTION OF THE FIGURES

To help a better understanding of the invention, a set of figures is included as an integral part of this description, where, for illustrative and non-limiting purposes, graphs of the experimental results obtained in the following examples are presented in this document.

**Figure 1.** Percentage of cell viability after 24 hours of exposure to different concentrations of the composition of the invention by MTT assay in cell lines 3T3 (figure 1.a) and HaCaT (figure 1.b). The bars represent the mean of six technical replicates and the error bars correspond to the standard deviation. The negative control (C-) represents a condition in which the cells were not exposed to any compound.

**Figure 2.** Secretion of the cytokines IL-8 (figure 2.a) and TNF-$\alpha$ (figure 2.b) by LPS-stimulated THP-1 cells under different concentrations of the composition of the invention in the presence of the *Lipopolysaccharides* (LPS) stimulus. The bars represent the mean of three technical replicates and the error bars correspond to the standard deviation. The negative control (C-) represents a condition in which the cells were not exposed to any compound. The positive control (C+) represents a condition in which the cells were exposed to the LPS stimulus.

**Figure 3.** Proliferation assay. Percentage of cell viability after 72 hours of exposure to different concentrations of the composition of the invention measured by MTT assay in human dermal fibroblasts (HDF). The bars represent the mean of six technical replicates and the error bars correspond to the standard deviation. The negative control (C-) represents a condition in which the cells were not exposed to any compound. The positive control (C+) represents a condition in which the cells were cultured with media supplemented with 10% Fetal Bovine Serum (FBS).

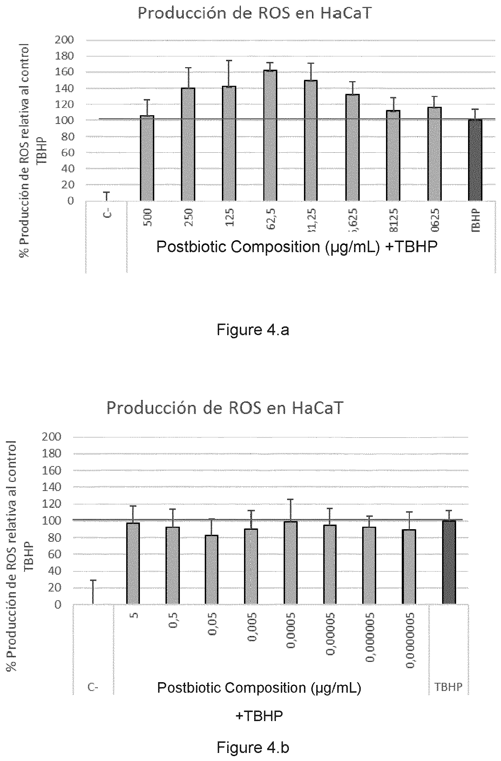

**Figure 4.** Percentage antioxidant activity of the test compound measured by the in vitro H2DFFDA assay in the HaCaT cell line. The results are presented in different concentration ranges, and are from 500 $\mu$g/mL to 3.9 $\mu$g/mL (figure 4.a) and from 5 $\mu$g/mL to 0.0000005 $\mu$g/mL (figure 4.b). The bars represent the mean of six technical replicates and the error bars correspond to the standard deviation. The negative control (C-) represents a condition in which the cells were not exposed to any product. Tert-butyl hydroperoxide (TBHP) is the Reactive Oxygen Species (ROS)-generating agent.

**Figure** 5. Representative image of the tail tip region showing the measured region (arrow).

**Figure** 6. Results obtained in the skin irritation assay. NC= negative control, PC = positive control.

**Figure** 7. Graphic plot and data representations of the cytotoxicity assay of the dilutions of the postbiotic composition of the invention. (A) and SDS (B)

**Figure** 8. Viability curves obtained in the phototoxicity assay. Postbiotic Composition (A) and reference item CPZ (B)

## EXAMPLES

So as to contribute to a better understanding of the invention, and in accordance with a practical embodiment thereof, a preferred embodiment of the present invention is attached hereto as an integral part of this description.

**EXAMPLE 1: STUDY TO DETERMINE THE THERAPEUTIC EFFICACY OF THE COMPOUND OF THE INVENTION BY IN VITRO STUDIES.**

First, an MTT cytotoxicity study was performed to determine the most appropriate concentrations of the composition of the invention to work with. Then, to evaluate the anti-inflammatory, proliferative and antioxidant activities of the compositions, specific in vitro assays were performed.

**MATERIALS Y METHODS**

Materials

The following reagents were used:

- Primary detachment kit. Innoprot, P6030.
- Newborn calf serum (NBCS). Gibco, 16010.159.
- Fetal bovine serum (FBS). Gibco, 10270-106.
- Hank's Balanced salts. Sigma, H-2387.
- L-glutamine 200 mM. Sigma, G7513.
- Penicillin/streptomycin (P/S). Gibco 15140-122.
- Trypsin-EDTA (10X). Sigma T4174.
- PBS; Phosphate-buffered saline (10X). ROCHE 11666789001.
- MycoAlert® Mycoplasma Detection Kit. Lonza LT07-318.
- Dubelcco's Modified Dubelcco's Minimum Essential Medium (DMEM). Sigma, D6648-10X.
- Fibroblast Culture Medium + Supplements. Innoprot, P60108
- RPMI; Roswell Park Memorial Institute -1640. Sigma, R0883.
- Human IL-8 ELISA kit Enzyme Linked Immunosorbent Assay. BD, 555244.
- Human TNF alpha ELISA kit. Invitrogen, EH3TNFA2.
- Sodium dodecyl sulphate (SDS). CAS No. 151-21-3 Sigma, L4509.
- E. coli lipopolysaccharide (LPS). Sigma, L3012.
- PMA (phorbol 12-myristate 13-acetate). Sigma, P1785.
- MTT. CAS No 298-93-1. Sigma; M2128.
- Carboxy-H2DFFDA. INVITROGEN; C-13293.
- Luperox® TBH70X, tert-butyl hydroperoxide solution (TBHP). SIGMA; 458139.

The following solutions were prepared:

- Hank's solution, without Ca2++ and Mg2++.
- MTT solution (50 $\mu$g/ml in DMEM).

Equipment

All equipment was calibrated following the quality standards according to UNE-EN ISO 9001, UNE-EN ISO/-IEC:17025 and GLP (Good Laboratory Practice):

- Automatic pipettes.
- Analytical Balance
- Multichannel pipettes.
- Thermostatic bath with shaking capacity.
- Freezer cabinet -80°C.
- Refrigerator.
- $CO_2$ incubator.
- Laminar flow Hoods.
- Varioskan microplate reader (Fluorimeter and spectrophotometer).
- Inverted optical microscope.
- Haemocytometer.
- Microplate shaker.
- Microcentrifuge.
- UV simulator
- ELISA plate reader

Postbiotic composition of the invention:

A postbiotic composed of lysates from 12 strains/species of *Lactobacillus sp., Bifidobacterium sp., Bacillus sp, Saccharomyces sp.* and *Streptococcus sp.* was used. The composition of the extracts appears in Table 2 in an amount in percentage by weight over the total weight of the lysates of the composition.

Table 2: Components of the postbiotic composition of the invention

| Species/Strains | % (w/w) |
|---|---|
| *Bacillus coagulans* | 3 |
| *Bacillus licheniformis* | 4 |
| *Bacillus mesentericus* | 4 |
| *Bacillus subtilis* | 4 |
| *Bifidobacterium lactis* | 5 |
| *Lactobacillus acidophilus* | 4 |
| *Lactobacillus bulgaricus* | 4 |
| *Lactobacillus casei* | 5 |
| *Lactobacillus paracasei* | 5 |
| *Lactobacillus rhamnosus* | 3 |
| *Saccharomyces cereviseae* | 56 |
| *Streptococcus thermophilus* | 3 |

Unless otherwise specified, the composition of Table 2 was used in all experiments.

Preparation of the different concentrations of the compound of the invention

All concentrations tested were prepared fresh on the day of the assay, directly in the culture medium required for each cell line.

Culture conditions and experimental systems for in vitro assays

Four experimental systems were used in this experiment: primary human dermal fibroblasts (HDF) and the THP-1, 3T3 and HaCaT cell lines. These cell lines were stored in the cell culture bank and tested for the absence of contaminating mycoplasmas immediately after thawing. The MycoAlert® mycoplasma detection kit was used for this purpose. By measuring the ATP level in the sample before (reading A) and after addition of the MycoAlertTM substrate (reading B), a ratio indicative of presence (Ratio > 1) or absence of mycoplasma contamination (Ratio < 0.9) is obtained.

HDF

Human Dermal Fibroblasts (HDF) purchased from Innoprot (P10858) were isolated by ScienCell Research Laboratories from human dermal tissue. HDF were grown in the medium of Fibroblasts + Supplements in standard culture conditions according to the manufacturer's instructions. At approximately 80-90% confluence, cells were trypsinised with the primary detachment kit, diluted at a 1:4 ratio, and subcultured. Cells subcultured 2-6 times were used in this study. Cell cultures were maintained under standard cell culture conditions (37°C, 5% $CO_2$ and 95% humidity).

HaCaT

The experimental system used for the study of the antioxidant efficacy was that of immortalized human keratinocytes (HaCaT cell line). This cell line was originally supplied by the Cell Line Service (CLS, Eppelheim, Germany). The cell line was maintained in complete culture medium (DMEM + 10% FBS) according to the supplier's instructions and routinely cultured in monolayer in tissue culture flasks. When cells exceeded 70% confluence (but less than 90%), they were subcultured into new culture flasks. All cell cultures were maintained under standard cell culture conditions (37°C, 5% $CO_2$ and 95% humidity).

THP-1

The experimental system used for the study of anti-inflammatory efficacy was THP-1 cell line (human monocyte cell line, ATCC® TIB-202). These cells were stored in the Gaiker cell culture bank and tested for the absence of contaminating mycoplasmas immediately after thawing. THP-1 cells are grown in suspension at 37°C in a humidified

atmosphere of 5% $CO_2$. Cells were maintained in culture medium (RPMI + 10 % FBSi + 50 $\mu$M) $\beta$-mercaptoethanol), for a minimum of two weeks prior to any experiment. Cells were subcultured before reaching 80% confluence (cell concentration/volume).

A cell suspension of $1 \times 10^5$ cells/mL was prepared 24 hours prior to the anti-inflammatory efficacy assay. $1 \times 10^4$ cells/well were dispensed into 96-well plates in the presence of 0.31 $\mu$g/mL PMA to differentiate monocytes to macrophages. Plates were incubated at 37°C, 5% $CO_2$ for 24 hours.

BALB/c 3T3

Adherent BALB/c 3T3 cells (mouse fibroblast cell line, ATCC® CCL-163TM) were cultured at 37°C in a humidified atmosphere of 5% $CO_2$. The culture medium was DMEM supplemented with 5% FBS and 5% Newborn Calf Serum (NCBS), non-essential amino acids and sodium pyruvate. Cells were subcultured before reaching 80% confluence (cell concentration/volume).

Cytotoxicity study

An MTT viability assay was performed to investigate the cytotoxicity of the postbiotic composition of the invention. Cells cultured in 96-well plates were treated with 8 concentrations of the test products for 24 hours (37°C, 5% CO2). In parallel, 8 concentrations of a positive control (sodium dodecyl sulphate (SDS)) were tested to validate the assay. After 24 hours of incubation with the test products or the positive control, the cells were washed with PBS, stained with MTT solution and incubated at 37°C for 2 hours. At the end of this incubation period, the staining medium was removed and 100 $\mu$l DMSO/well was added to solubilize the colored precipitate. Absorbance was read at 540 nm on the plate reader of the spectrophotometer.

The percentage of cell viability was calculated relative to the negative control, a condition in which the cells were not exposed to any product.

$$\% \text{ cell viability} = AbT/AbC \times 100$$

AbT = absorbance at 540 nm after 72 hours of treatment

AbC = absorbance at 540 nm after 72 hours without treatment (negative control).

Anti-inflammatory assay

For the anti-inflammatory assay, PMA-differentiated THP-1 cells were incubated with the test products at different concentrations for 24 hours in the presence of the inflammatory stimulus lipopolysaccharide, LPS (20 $\mu$g/ml). Non-inflamed cells (culture medium) were used as negative control. Cell secretion of IL-8 and TNF-$\alpha$ was measured by ELISA.

Antioxidant activity study: Detection of ROS by H2DFFDA

Antioxidant activity was studied by H2DFFDA, a method used to determine the production of reactive oxygen species (ROS). Cells were cultured in 96-well plates and incubated with H2DFFDA for 45 minutes. After incubation, the cells were washed with PBS and exposed to tBHP (ROS-generating agent) in the presence or absence of the test products. After an incubation period of 4 hours, fluorescence was measured in a microplate reader (Ex=485 nm; Em=535 nm). The percentage of antioxidant activity (AA%) was calculated by comparing the fluorescence values of the control (negative control) and the test samples using the following formula

$$AA\% = 100 - \left[\frac{\left(F^{485/535}_{CX} - F^{485/535}_{B-CX}\right) \times 100}{F^{485/535}_{Control}}\right]$$

$F^{485/535}$ **Cx:** Fluorescence at Ex/Em = 485/535 nm of the postbiotic composition of the invention at a given concentration exposed to H2DFFDA and tBHP.

$F^{485/535}$ **B-Cx**: Fluorescence at Ex/Em = 485/535 nm of the test product at a given concentration exposed to tBHP but not to H2DFFDA (blank).

**F**[485/535] **Control:** Fluorescence at Ex/Em = 485/535 nm of the test buffer exposed to H2DFFDA and tBHP (C-).

## EXAMPLE 2: TISSUE REGENERATION IN A ZEBRA FISH MODEL

The main objective of this study was to evaluate the efficacy of the postbiotic composition of the invention (administered in the diet) in promoting tissue regeneration of the caudal fin after amputation

Adult zebrafish were housed and maintained following standard procedures. Briefly, fish were maintained in 3-liter aquaria heated to 28.5 $_\circ$C, with about 20 fish per tank and water continuously filtered. Water was maintained at pH 7-7.8, conductivity at 500-800 $\mu$S, and $O_2$ saturation between 80-100%. Water conditions were monitored and regulated conveniently. Fish were kept under a photoperiod of 14:10 hours light:dark. Adults were fed with ground dry pellets and live food, following a regular practice in the field.

Embryos were raised in E3 media with ampicillin (100 $\mu$g/ml) and methylene blue (0.0001%), and kept in plates in the incubator at 28.5 °C until they reached 5 dpf (days post fertilization). At that time, they were transferred to 3-liter tanks until the day of the experiment. From 5 dpf onwards, these larvae were fed with their corresponding experimental diets (standard and supplemented diets) on a daily basis (3 times a day) until the end of the experiment. The day of amputation larvae were fed only once.

Sample size: 30 larvae per experimental group, distributed in 2 nests (15 larvae/nest, 2 nests per 3-liter tank).

- two experimental groups (each group in one tank).

- The following aquariums (tanks) were randomly distributed in the stand alone

  - Group 1: Standard diet (negative control).

  - Group 2: Supplemented diet (50% Standard diet + 50% postbiotic composition of the invention).

- Feeding with the test diets took place from 5 dpf to 15 dpf, 3 times per day except on the day of amputation (12 dpf) when they were fed only once

- Partial amputation of the caudal fin was done at 12 dpf and pictures were taken at 15 dpf. The length of the regenerated tissue was measured.

### Larvae production and housing

Synchronized embryos were supplied to the laboratory and kept at 28.5 °C in an incubator until they reached 5 dpf. At this time, larvae were transferred to 3-liter tanks and fed manually. An automatic water recirculation system with a daily replacement of 10% of water was employed until 12 dpf.

### Diet preparation and feeding regimen

Larvae were fed with their respective diets three times a day (except on the day of amputation when they were fed only once) with a total amount of 18 mg/day and tank. A standard diet consisted of powder zebrafish larva food (Sera Micron). The supplemented diet consisted of 50% standard diet plus 50% of the composition of the invention and was prepared by thorough mixing of the powders.

All diets were spray-coated with vegetal oil and left to dry overnight at room temperature in a dry and dark place. Diets were kept at 4 °C during the duration of the trial to avoid degradation of the test compounds. A total amount of 792 mg of Sera Micron and a mix of 396 mg of Sera Micron plus 396 mg of the postbiotic composition of the invention were initially prepared for the standard diet and the supplemented diet, respectively.

### Tail amputation, treatment and analysis

At 12 dpf, larvae from all experimental groups were anesthetized with tricaine and the tail fin was partially amputated with a sterile razor. Bright field and fluorescent pictures (using Leica stereo microscope) of the injured site were taken just before the amputation and at 15 dpf. The fish were anesthetized and immersed in 3% Methylcellulose before imaging. After that, larvae were placed back to water system without Tricaine and once recuperated were transferred to their respective tanks to continue with the experiment.

The length of the regenerated tissue at 15 dpf was measured and a comparison during the regeneration time between the two experimental groups was made.

## EXAMPLE 3: SKIN IRRITATION ASSAY AND PHOTOTOXICITY ASSAY

The test system used in skin irritation assay was the RHE model (Reconstructed Human Epidermis), provided by Skinethic (Reference RHE/S/17). Tissues were exposed to the postbiotic composition of the invention for 42 minutes. Then, each tissue was thoroughly rinsed and transferred to fresh medium, where they were incubated for another $42 \pm 4$ hours. Afterwards, the cell viability of the treated tissues was determined, by the MTT assay.

The phototoxicity assay used 3T3 cells as test system. The aim of this study was to obtain the Photo-Irritation Factor (PIF) for the postbiotic composition of the invention. 3T3 cells were treated with the postbiotic composition of the invention at eight different concentrations for 1 hour. Thereafter, half of the samples were exposed to simulated solar irradiation, whereas the other half was kept in the dark. Cell viability was calculated after 24 hours of incubation by means of the neutral red assay. The following reagents were used for these assays. Table 3

Table 3 Reagents used in this example

| SKIN IRRITATION | |
|---|---|
| **Reagent** | **Reference** |
| MTT | Sigma M2128-1G |
| Isopropanol | MTT-100-EXT |
| 5% SDS (positive control) | TC-5%-SDS |
| dPBS (10X) | Sigma D1408-500ML |
| dPBS | TC-PBS |
| Culture media (maintenance) | 21 SMM 049 |
| Culture media (growth) | 21 SGM 135 |
| PHOTOTOXICITY | |
| MTT | Sigma M2128-1G |
| PBS | Roche 11666789001 |
| SDS | Sigma L6026 |
| Tripan Blue | Gibco 15250-061 |
| DMEM | Sigma D5648 |
| Penicillin/Streptomycin | Gibco 141540-122 |
| NaHCO$_3$ | Sigma 58875-500G |
| Hank's salt with phenol red | Sigma H2387 |
| Hank's salt without phenol red | Sigma H1387 |
| Tripsina 10X | Sigma T4174 |
| DMSO | Panreac 131954.1616 |
| Acetic acid | Fisher 161954.161612 |
| New born calf serum (NCBS) | Gibco 16010-159 |
| Neutral red | Sigma N2889-20ml |
| Ethanol | Panreac 131085.1212 |
| Acetic acid | Panreac 131008.1611 |
| SDS | Sigma-Aldrich L6026 |
| CPZ | Sigma-Aldrich C0982-5G |
| HEPES | Sigma-Aldrich H3375 |
| NaOH 1M | 1.09137.1003 |

The same equipment used in the previous example was also employed for the skin irritation and phototoxicity assays

The following solutions were prepared: dPBS 1X, MTT 1 mg/m, DMEM supplemented + 10% NBCS, HBSS with and without Ca2+ or Mg2+, Trypsin 1X, HEPES 1M, Trypan blue, DMEM supplemented + 10% NBCS + 5% DMSO, DMEM supplemented + 10% NBCS + 25mM HEPES, Neutral red solution and Neutral red desorb solution.

*Test systems: in vitro skin irritation assay*

The RHE model was used as a test system. This model consists of normal human-derived epidermal keratinocytes, which have been cultured from a multilayered highly differentiated model of the human epidermis. It consists of organized basal, spinous and granular layers, and a multilayered stratum corneum containing intercellular lamellar lipid layers arranged in patterns analogous to those found in vivo. To prepare the system, on the day of the receipt, tissues were conditioned by incubation with the assay medium to release transport stress related compounds and debris overnight.

*Test systems: in vitro phototoxicity assay*

Balb/c 3T3 cells were used as a test system. Once thawed, cells were grown as a monolayer in DMEM culture medium supplemented with 10% Newborn Calf Serum (NCBS) and antibiotics (100 U/mL penicillin and 100 μg/mL streptomycin). The cells were incubated under standard culture conditions (37°C $\pm$ 2°C and 5% CO2/air), changing the medium every 2-3 days and sub-cultivating before reaching 80% confluency. According to the guideline, the 3T3 cells used in this study were tested for mycoplasma and only mycoplasma-free cells were used throughout the study.

To prepare the system, after thawing, cells were propagated from stock cultures and grown in culture medium for two weeks. For each assay, when cell density reached 80% confluence, they were trypsinized and counted using trypan blue strain. The day before the exposition to the postbiotic composition of the invention, the cells were tripsinized and counted. A suspension of 100,000 cells/mL was prepared and 200 μL of the suspension were added to each well in 96-well plates (20,000 cells/well), except in the peripheral wells.

*Preparation of the postbiotic composition of the invention*

Each assay required different concentrations of the postbiotic composition.

**[0149]** - The skin irritation assay uses the composition as neat, so no preparation was needed. The protocol for the skin irritation assay is disclosed in OECD Guideline for testing of chemicals: In vitro Skin Irritation: Reconstructed Human Epidermis Test Method, protocol No 439 (2019)

To prepare the system, on the day of the receipt, tissues were conditioned by incubation with the assay medium to release transport stress related compounds and debris overnight. The inserts containing reconstituted human epithelium were placed onto 1 mL of the SGM medium in 6-well plates. The plates were incubated overnight at 37 °C, 5% CO2.

The next day, the tissues were transferred to 24-well plates containing 300 μL of SMM medium. 10 μL of sterile water was added to the top of the tissues and then approximately 16 mg of the test postbiotic composition of the invention were added to each issue. 16 μL of the negative control (dPBS) and positive control (SDS 5%) were applied to the corresponding tissues. After dosing the last tissue, all the inserts were incubated for 42 minutes at room temperature. At the end of the exposure, the tissues were rinsed with dPBS. The inserts were then incubated with 2 mL of SGM medium for 42 hours under standard culture conditions. The MTT assay was carried as disclosed previously in example 1, the relative viability was obtained from the Optical Density values. OD values were corrected by subtracting the blank values (isopropanol). Then, the relative cell viability was calculated as follows: Relative viability (%) = OD postbiotic composition / mean OD negative control *100.

**[0152]** - Prior to the phototoxicity assay, solubility of the composition was determined, it was decided to start from 20 mg/mL in culture media. Starting from this concentration, a dose range finding experiment (cytotoxicity assay) was performed. Starting from 20 mg/mL in culture media, eight concentrations were prepared in 1/10 dilutions: 20 mg/mL; 2 mg/mL; 0.2 mg/mL; 0.02 mg/mL; 0.002 mg/mL; 0.0002 mg/mL; 0.00002 mg/mL; and 0.000002 mg/mL

According to the OECD Guideline for testing of chemicals: In Vitro 3T3 NRU phototoxicity test, protocol No 432 (2019), the maximum concentration to be analyzed is 1 mg/mL. As the cytotoxicity assay exposed the cells to the composition for 24 hours, and the phototoxicity assay includes an exposition of 1 hour, it was decided to perform the phototoxicity test starting from 1 mg/mL. From this concentration, eight concentrations were prepared in 1/3 dilutions. This solution was prepared in Hank's with Ca2+ and Mg2+ and HEPES. (C1) 1 mg/mL; (C2) 0.33 mg/mL; (C3) 0.11 mg/mL; (C4) 0.037 mg/mL; (C5) 0.0123 mg/mL; (C6) 0.0041 mg/mL; (C7) 0.00137 mg/mL; and (C8) 0.000457 mg/mL SDS was used as a positive control, 8 concentrations were prepared 400 μg/mL; 200 μg/mL; 100 μg/mL; 50 μg/mL; 25 μg/mL; 12.5 μg/mL; 6.25 μg/mL; and 3.125 μg/mL

Starting from the limit of solubility obtained (20 mg/mL) a dose range finding experiment was set up in order to

determine the $IC_{50}$ value (if obtained). 3T3 cells were seeded at 10,000 cells/well in 96-well plates. Seven more concentrations were prepared following a 1/10 dilution factor. Cells were exposed to the postbiotic composition of the invention for 24h. SDS was used as positive control and viability was determined by means of MTT method.

The cytotoxicity assay showed toxicity signs up to 2 µg/mL. As the exposition time involved in the phototoxicity test was shorter, it was decided to start this assay from highest concentration allowed: 1 mg/mL. Seven more concentrations were prepared in 1/3 dilutions. 3T3 cells were seeded at 20,000 cells/well in 96-well plates. Cells were exposed to the eight concentrations of the postbiotic composition of the invention defined above for 1 hour at 37°C and 5% CO2. Two 96-well plates were prepared for the postbiotic composition of the invention, the postbiotic composition and reference control (CPZ). One plate of each condition (+IRR) was irradiated for 52 minutes at 5 J/cm$^2$ while the other plates (- IRR) were kept in the dark for the same amount of time. Then, culture media was removed, cells were washed with Hank's without phenol red and 100 µL of neutral red were added to each well. Cells were incubated for 3 hours at 37°C and 5% $CO_2$ and then were washed again. Finally, the neutral red was solubilized by adding 100 µL to each well of a solution of Acetic acid/Ethanol. Plates were incubated 10 minutes at room temperature and absorbance at 540 nm was measured.

## RESULTS

Mycoplasma Assay

The results obtained in the mycoplasma test are shown in Table 4:

Table 4: mycoalert ratio obtained from cell culture

| Cells: | Measurement A | Measurement B | Ratio (B/A) |
|---|---|---|---|
| THP-1 | 1183 | 723,1 | 0.611 |
| 3T3 | 1906 | 953.0 | 0.500 |
| HaCaT | 702.9 | 355.7 | 0.506 |
| HDF | 1451 | 882.0 | 0.608 |

The ratios obtained in the MycoAlert® Mycoplasma Detection Kit for all cell lines were below 0.9, indicating that the cells were not contaminated with mycoplasma. Therefore, all experimental systems were in perfect condition to perform all assays.

Cytotoxicity study

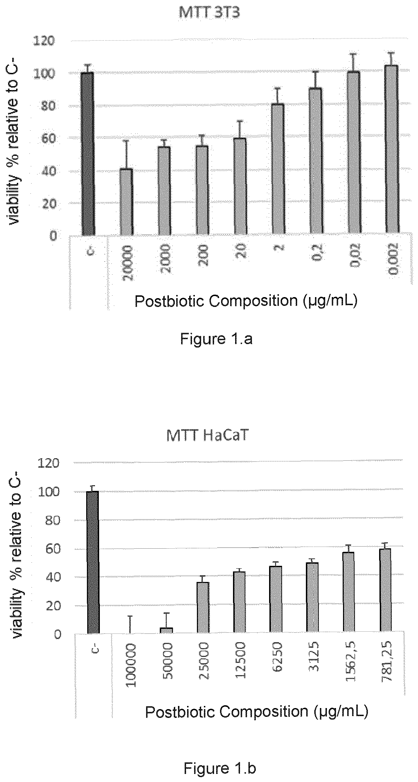

The cytotoxic effect of the postbiotic composition of the invention was tested in an in vitro assay using the MTT assay on HaCaT and 3T3 cell lines. Eight concentrations of the test compound were tested: 20,000 µg/mL, 2,000 µg/mL, 200 µg/mL, 20 µg/mL, 2 µg/mL, 0.2 µg/mL, 0.02 µg/mL and 0.002 µg/mL or 100,000 µg/mL, 50. 000 µg/mL, 25,000 µg/mL, 12,500 µg/mL, 6,250 µg/mL, 3,125 µg/mL, 1562.5 µg/mL and 781.25 µg/mL and were tested on the 3T3 and HaCaT cell lines, respectively. In parallel, different concentrations of sodium dodecyl sulphate (SDS) were used as a positive control (not shown). The results are shown in Figure 1 and Table 5.

Table 5: Cytotoxicity of the postbiotic composition

| 3T3 MTT | | Postbiotic composition of the invention (µg/mL) | | | | | | | |
|---|---|---|---|---|---|---|---|---|---|
| Replicates | c- | 20000 | 2000 | 200 | 20 | 2 | 0,2 | 0,02 | 0,002 |
| 1 | 107,14 | 8,32 | 50,31 | 54,74 | 67,49 | 83,49 | 96,97 | 103,95 | 106,03 |
| 2 | 99,2 | 36,7 | 53,92 | 50,86 | 58,01 | 70,37 | 87,53 | 84,09 | 89,39 |
| 3 | 103,62 | 55,56 | 53,02 | 64,01 | 68,52 | 77,45 | 85,75 | 108,33 | 111,75 |
| 4 | 93,33 | 52,76 | 61,27 | 57,01 | 65,17 | 98,04 | 99,38 | 105,19 | 102,59 |
| 5 | 97,76 | 43,21 | 57,2 | 55,9 | 55,73 | 75,75 | 94,59 | 107,59 | 101,56 |
| 6 | 98,94 | 49,08 | 48,6 | 44,79 | 39,86 | 75,16 | 72,14 | 85,24 | 106,77 |

(continued)

|  |  | Postbiotic composition of the invention (µg/mL) | | | | | | | |
|---|---|---|---|---|---|---|---|---|---|
|  | c- | 20000 | 2000 | 200 | 20 | 2 | 0,2 | 0,02 | 0,002 |
| Average | 100 | 40,94 | 54,05 | 54,55 | 59,13 | 79,97 | 89,39 | 99,06 | 103,02 |
| Standard Deviation | 4,8 | 17,36 | 4,62 | 6,42 | 10,76 | 9,75 | 9,98 | 11,27 | 7,58 |
| HaCaT MTT |  | Postbiotic composition of the invention (µg/mL) | | | | | | | |
| Replicates | c- | 100000 | 50000 | 25000 | 12500 | 6250 | 3125 | 1563 | 781 |
| 1 | 98,83 | -11,85 | 3,68 | 39,94 | 45,47 | 43,77 | 48,35 | 59,45 | 60,14 |
| 2 | 101,99 | -10,51 | 23,53 | 41,52 | 45,2 | 50,57 | 52,01 | 61,66 | 61,92 |
| 3 | 96,26 | -24,31 | 1,21 | 34,94 | 42,39 | 43,1 | 47,08 | 48,89 | 52,65 |
| 4 | 101,34 | -36,29 | -8,47 | 36,74 | 40,54 | 44,9 | 45,3 | 50,14 | 53,44 |
| 5 | 105,86 | -35,23 | 1,03 | 30,78 | 42,61 | 50,27 | 52,22 | 57,01 | 60,46 |
| 6 | 95,71 |  | 1,47 | 29,66 | 39,38 | 44,96 | 46,24 | 55,53 | 59,11 |
|  |  | Postbiotic composition of the invention (µg/mL) | | | | | | | |
|  | c- | 100000 | 50000 | 25000 | 12500 | 6250 | 3125 | 1563 | 781 |
| Average | 100 | 0 | 3,74 | 35,6 | 42,6 | 46,26 | 48,54 | 55,45 | 57,95 |
| Standard Deviation | 3,84 | 12,31 | 10,58 | 4,78 | 2,44 | 3,3 | 2,95 | 5,06 | 3,92 |

Figure 1 shows that concentrations below 2 µg/ml resulted in cell viability of over 80% in the 3T3 experimental system. Concentrations close to 700 µg/mL and 20 µg/mL reduced cell viability to 60% in the HaCaT and 3T3 experimental systems, respectively. These concentrations were used for the following efficacy assays.

Anti-inflammatory efficacy

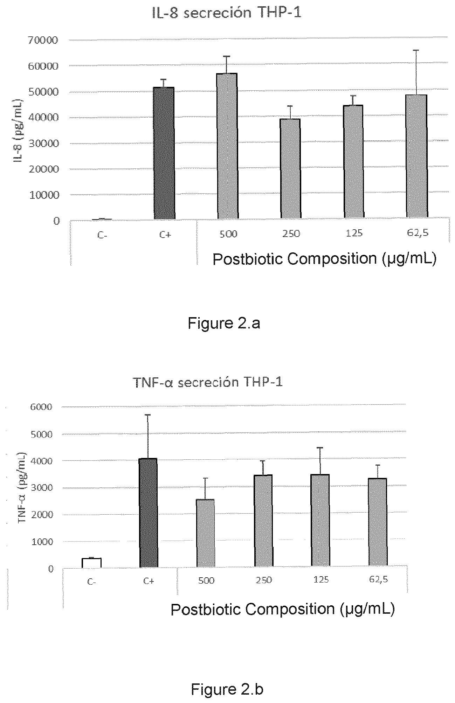

THP-1 cells differentiated into macrophages were simultaneously exposed to the inflammatory insult, LPS, and different concentrations of the postbiotic composition of the invention (selected according to cytotoxicity results) for 24 hours. After this exposure time, cell supernatants were collected and analysed. The anti-inflammatory effect of the test compound was analysed by quantifying the levels of IL-8 and TNF-$\alpha$ secretion by ELISA. The results are shown in Figure 2. The test compound showed a dose-dependent anti-inflammatory capacity at concentrations ranging from 250 µg/ml to 62.5 µg/ml.

Proliferative activity

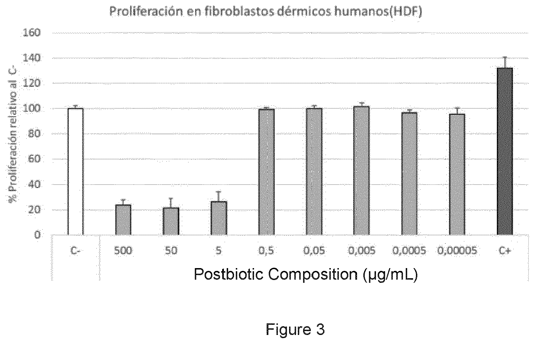

To further investigate the effect of the postbiotic composition of the invention on cell proliferation, an in vitro proliferation assay using MTT was performed on primary human dermal fibroblasts. As a positive control, cells were cultured in media containing 10% FBS. The results are shown in Figure 3. In general, the test compound shows no proliferative effect at concentrations ranging from 0.5 µg/ml to 0.00005 µg/ml. Concentrations above 0.5 µg/mL caused cell cytotoxicity, affecting cell viability.

Antioxidant activity

The antioxidant activity of the postbiotic composition of the invention was evaluated in an in vitro assay in the HaCaT cell line by measuring the inhibition of reactive oxygen species (ROS) production. All experimental groups, except the negative control, included TBHP (ROS-generating agent). Two independent assays were performed with different concentration ranges. From 500 µg/mL to 3.9 µg/mL and from 5 µg/mL to 0.0000005 µg/mL. The results are shown in Figure 4a, 4b and Table 6. The postbiotic composition of the invention shows antioxidant activity at concentrations ranging from 0.5 µg/mL to 0.005 µg/mL. The rest of the concentrations tested show no antioxidant activity (ROS).

Table 6. Antioxidant activity

| Replicates | C- | Postbiotic composition of the invention (μg/mL)+ TBPH | | | | | | | | C+(TBPH) |
|---|---|---|---|---|---|---|---|---|---|---|
| | | 500 | 250 | 125 | 62,5 | 31,25 | 15,625 | 7,8125 | 3,90625 | |
| 1 | -11,94 | 106,29 | 162,69 | 163,25 | 163,36 | 157,14 | 118,96 | 124,39 | 115,23 | 102,44 |
| 2 | -6,22 | 119,69 | 140,51 | 158,39 | 176,83 | 140,79 | 146,68 | 101,88 | 124,9 | 103,8 |
| 3 | 2,94 | 114,89 | - | 104,7 | 156,69 | 142,09 | 131,74 | 107,31 | 126,54 | 116,98 |
| 4 | 0,17 | 116,13 | 168,34 | - | 161,84 | 155,56 | 151,09 | 130,67 | 126,77 | 105,84 |
| 5 | -5,15 | 70,99 | 113,81 | - | - | 183,11 | 134,63 | 118,68 | 112,34 | 94,13 |
| 6 | 20,19 | - | 114,32 | - | 151,15 | 120,09 | 108,38 | 87,56 | 91,98 | 76,82 |
| | C- | Postbiotic composition of the invention (μg/mL)+ TBPH | | | | | | | | C+(TBPH) |
| | | 500 | 250 | 125 | 62,5 | 31,25 | 15,625 | 7,8125 | 3,90625 | |
| Average | 0 | 105,6 | 139,93 | 142,11 | 161,97 | 149,8 | 131,91 | 111,75 | 116,29 | 100 |
| Stand. Dev | 11,17 | 19,96 | 25,8 | 32,49 | 9,59 | 21,07 | 16,21 | 15,92 | 13,39 | 13,53 |

The postbiotic composition of the invention exhibits dose-dependent anti-inflammatory activity in the THP-1 cell line at concentrations ranging from 250 $\mu$g/mL to 62.5 $\mu$g/mL. The test compound shows no proliferative effect in primary human dermal fibroblasts at concentrations ranging from 0.5 $\mu$g/ml to 0.00005 $\mu$g/ml.

Tissue regeneration in a Zebra Fish model

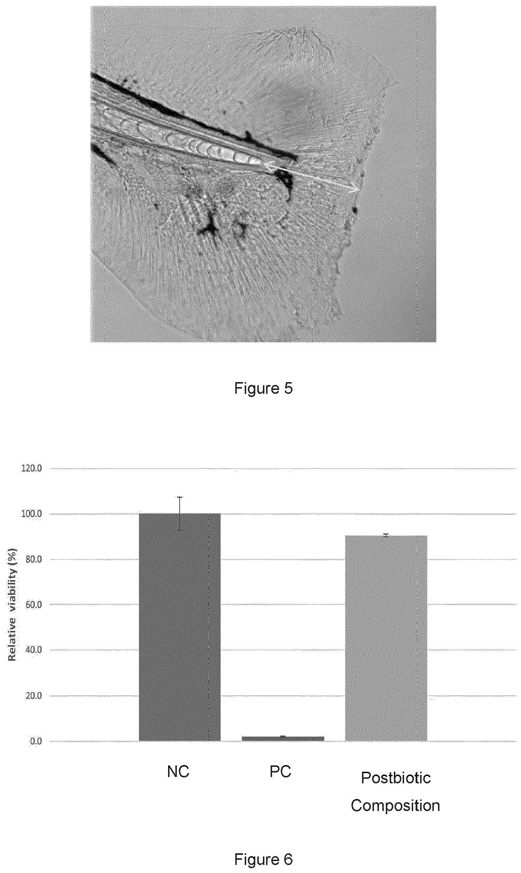

Three days later after the day of amputation (12 dpf) this is, 15 dpf, the length from the distal part of the notochord to the most distal part of the caudal fin was measured for each larva (as shown in Figure 5 and Table 7) by using the straight line and measuring tools of Imaged software.

Table 7. Mean value obtained for tail regrowth in all the experimental groups

| Tail Length Means at 15dpf (Mean) | |
|---|---|
| Standard diet | 0.173 mm |
| Supplemented diet | 0.185 mm |

The main objective of this Study was to assess the potential value of the postbiotic composition of the invention on the tissue regeneration capacity in a zebrafish tail resection model. For this purpose, the postbiotic composition was administered in the diet to zebrafish larvae from 5 to 15 dpf. A caudal fin amputation was performed on day 12 post-fertilization and the regrown tissue was analyzed using distance parameters. The postbiotic diet give a significant advantage on the regeneration pace of the amputated tails when compared with the standard diet groups from 15 dpf time point between Groups 1 and 2 (standard and supplemented diet respectively). If the tail regrowth measurement would have been done after 15 dpf, the difference between standard diet and the supplemented diet would have been larger. Furthermore, a higher amount than 18 mg/day and tank would show a greater difference between standard diet and supplemented diet in this experiment

Example 3: Skin irritation assay and Phototoxicity assay

*Skin irritation assay: OECD 439*

The results obtained in the skin irritation assay are shown in Fig. 6. An irritant is predicted if the mean relative tissue viability of three individual tissues exposed to the test item (postbiotic composition of the invention) is reduced below 50% of the mean of the negative controls. Table 8 shows the result obtained for the composition of the invention. The postbiotic composition of the invention is classified as Non-irritant (GHS No Category)

Table 8 % of viabilities

| | Mean of viabilities (%) | Classification |
|---|---|---|
| Negative Control (NC) | 100 | Non Irritant (NI) |
| Positive Control (PC) | 2.0 | Irritant (I) (R30 o GHS cat. 2) |
| Postbiotic Composition | 90.4 | Non Irritant (NI) |

*In vitro phototoxicity assay*

*Dose range finding*

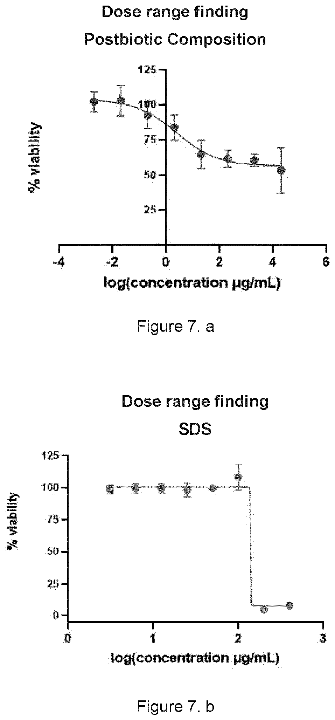

To establish the concentrations to be tested in the phototoxicity assay, it was decided to check the postbiotic composition of the invention in a dose range finding assay using SDS as a positive control. Starting from 20 mg/mL dissolved in culture media it was decided to prepare the concentrations disclosed above in the materials and methods section. Using the Graphpad Prism 8 statistical software, we calculated the IC50 values by means of a 4-parameter logistic curve (4PL). This equation uses log(dose) values. This model does not assume a standard slope, but rather fits the Hill Slope from the data figure 7. According to the results, the postbiotic composition of the invention showed an $IC_{50}$ of 2.44 $\mu$g/mL (value calculated from Graph Pad Prism). The SDS showed results within the known range.

*Phototoxicity assay*

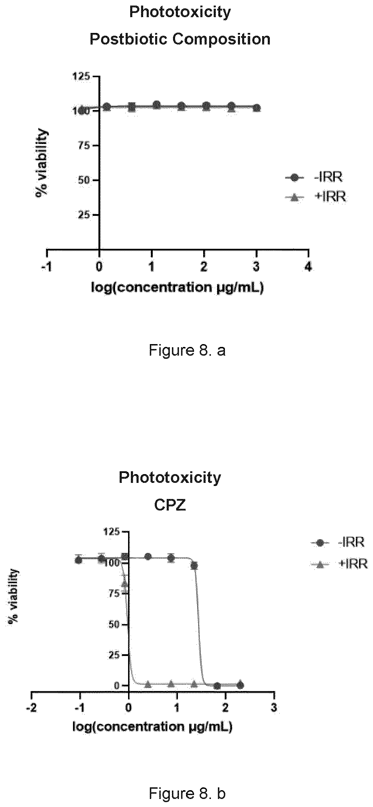

Once obtained the cytotoxic results, the phototoxicity assay for the postbiotic composition of the invention was performed using 1 mg/mL as the highest dose. Although toxic signs were observed at this concentration, the exposure time during cytotoxicity assay was longer than in the phototoxicity test. Therefore, it was decided to use the highest concentration allowed according to OECD 432 guideline. In total, 8 concentrations were prepared as disclosed in the material and methods section above. In parallel, 8 concentrations of the reference item Chlorpromazine (CPZ) were also prepared: (C1) 200 μg/mL; (C2) 66.67 μg/mL; (C3) 22.22 μg/mL; (C4) 7.407 μg/mL; (C5) 2.47 μg/mL; (C6) 0.823 μg/mL; (C7) 0.274 μg/mL; and (C8) 0.091 μg/mL. As it can be observed in the figure 8, and Table 9 the non-irradiated (-IRR) plates showed good results compared to the irradiated plates (+IRR) and $IC_{50}$ values could be calculated for CPZ.

Table 9 Viability curves obtained in the Phototoxicity assay

| % viability | | | | | | | | |
|---|---|---|---|---|---|---|---|---|
| | C1 | C2 | C3 | C4 | C5 | C6 | C7 | C8 |
| Postbiotic -IRR | 102.43% | 104.02% | 104.09% | 103.96% | 104.98% | 103.37% | 103.43% | 100.29% |
| Postbiotic +IRR | 102.34% | 101.95% | 103.06% | 103.02% | 104.38% | 102.65% | 102.75% | 101.69% |
| CPZ -IRR | 0.41% | 0.01% | 97.82% | 104.06% | 105.32% | 105.22% | 103.56% | 102.22% |
| CPZ +IRR | 2.57% | 0.32% | 1.62% | 1.75% | 1.31% | 83.43% | 103.77% | 103.42% |

According to the OECD 432 guideline, if an $IC_{50}$ cannot be calculated, a PIF value cannot be determined for the postbiotic composition of the invention. So, the mean photo effect (MPE) is calculated. In this study, the IC50 value for cells exposed to the postbiotic composition could not be determined, while the IC50 of CPZ was 31.406 for non-irradiated plates and 0.974 for irradiated plates. The calculation of the MPE and PIF values for controls and the postbiotic composition was performed with a software developed within the OECD 432 framework and available in http://www.oecd.org/env/ehs/tes ting/section4software.htm

To predict the phototoxic potential, the concentration responses obtained in the presence (+Irr) and in the absence (-Irr) of solar irradiation were compared and photo-irritation factor (PIF) and/or the mean photo effect (MPE) was calculated, results are shown in Table 10

Table 10 PIF (and MPE) calculation values

| Sample | PIF $\dfrac{IC50\ (-Irr)}{IC50\ (+Irr)}$ | MPE $\dfrac{\sum_{i=1}^{n} w_i\, PE_{ci}}{\sum_{i=1}^{n} w_i}$ |
|---|---|---|
| **Postbiotic** | Not available | 0.005 |
| **CPZ** | 32.303 | 0.401 |

According to the criteria defined in the "OECD guideline for Testing of Chemicals, Protocol No. 432 the results obtained should meet the following criteria: After the irradiation with UVA dose of 5 J/cm$^2$ the cell viability of the solvent control was > 80% of non-irradiated cells. In this experiment, the % difference between solvent controls - mean solvent controls (this is, non-irradiated plates) was 1.61% for the postbiotic composition and 2.74 % for CPZ. The PIF value for the positive reference (CPZ) is disclosed in the previous table. According to these results, the postbiotic composition of the invention is classified as non-phototoxic

**Claims**

1. A postbiotic composition that comprises lysates of probiotic microorganisms in a percentage amount by weight with respect to the total weight of the lysates of microorganisms of the composition:

    - 6% to 19% of bacterial lysates of the genus *Bacillus*;
    - 4% to 8% of bacterial lysates of the genus *Bifidobacterium*;
    - 15% to 25% of bacterial lysates of the genus *Lactobacillus*;

- 50% to 60% of yeast lysates of the genus *Saccharomyces*;
- 1.5% to 7% of bacterial lysates of the genus *Streptococcus.*

for use in the treatment of skin disorders.

2. The composition for use, according to the preceding claim, wherein the bacterial lysates of the genus *Bacillus* are of the species that are selected from the group consisting of *Bacillus clausii, Bacillus coagulans, Bacillus licheniformis, Bacillus pumilus, Bacillus subtilis, Bacillus mesentericus Bacillus paralicheniformes,* and combinations thereof.

3. The composition for use, according to any of the preceding claims, wherein the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof.

4. The composition for use, according to any of the preceding claims, wherein the bacterial lysates of the genus *Lactobacillus* are of the species that are selected from the group consisting of *Lactobacillus lactis, Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus Lactobacillus salivarius, Lactobacillus helvéticus, Lactobacillus gasseri, Lactobacillus johnsonii, Lactobacillus brevis, Lactobacillus kefiri,* and combinations thereof.

5. The composition for use, according to any of the preceding claims, wherein the lysates of yeast genus *Saccharomyces* are of the species that are selected from the group consisting of *Saccharomyces cerevisiae, Saccharomyces boulardi,* and combinations thereof.

6. The composition for use, according to any of the preceding claims, wherein the bacterial lysates of the genus *Streptococcus* are of the species that are selected from the group consisting of *Streptococcus thermophilus, Streptococcus salivarius,* and combinations thereof.

7. The composition for use, according to any of the preceding claims, wherein the percentage by weight of *Bacillus licheniformis, Bacillus mesentericus* and *Bacillus subtilis* is approximately the same, and is higher than that of *Bacillus coagulans.*

8. The composition for use, according to any of the preceding claims, wherein the bacterial lysates of the genus *Lactobacillus* are of the species *Lactobacillus acidophilus, Lactobacillus casei, Lactobacillus paracasei, Lactobacillus bulgaricus, and Lactobacillus rhamnosus,* and the percentage by weight of *Lactobacillus casei,* and *Lactobacillus paracasei* is approximately the same, and is higher than that of *Lactobacillus acidophilus* and *Lactobacillus bulgaricus,* which is also approximately the same and is higher than that of *Lactobacillus rhamnosus.*

9. The composition for use, according to any of the preceding claims, wherein the composition comprises lysates of probiotic microorganisms in an amount in percentage by weight of the total lysates of the composition:

   - 6% to 19% of bacterial lysates of *Bacillus licheniformis, Bacillus coagulans, Bacillus mesentericus* and *Bacillus subtilis;*
   - 4% to 8% of bacterial lysates of *Bifidobacterium lactis;*
   - 15% to 25% of bacterial lysates of *Lactobacillus acidophilus, Lactobacillus casei, Lactobacillus bulgaricus, Lactobacillus paracasei,* and *Lactobacillus rhamnosus*;
   - 50% to 60% of yeast lysates of *Saccharomyces cerevisiae*;
   - 1.5% to 7% of bacterial lysates of *Streptococcus thermophilus.*

10. The composition for use, according to any of the preceding, wherein the composition comprises lysates of probiotic microorganisms in an amount in percentage by weight of the total lysates of the composition:

   - 6% to 19% of bacterial lysates of *Bacillus licheniformis, Bacillus coagulans, Bacillus mesentericus* and *Bacillus subtilis,* the percentage by weight of *Bacillus licheniformis, Bacillus mesentericus* and *Bacillus subtilis* is the same, and is higher than that of *Bacillus coagulans.*
   - 4% to 8% of bacterial lysates of *Bifidobacterium lactis;*
   - 15% to 25% of bacterial lysates of *Lactobacillus acidophilus, Lactobacillus casei, Lactobacillus bulgaricus,*

*Lactobacillus paracasei,* and *Lactobacillus rhamnosus,* the percentage by weight of *Lactobacillus casei,* and *Lactobacillus paracasei* is the same, and is higher than that of *Lactobacillus acidophilus* and *Lactobacillus bulgaricus,* which is also the same and is higher than that of *Lactobacillus rhamnosus.*
- 50% to 60% of yeast lysates of *Saccharomyces cerevisiae*;
- 1.5% to 7% of bacterial lysates of *Streptococcus thermophilus.*

11. The composition for use, according to any of the preceding claims, wherein the skin disorders are oxidative stress and/or inflammatory diseases of the skin.

12. The composition for use, according to the preceding claim, wherein the skin disorders are one or more of the following disorders: eczema, psoriasis, acne, rosacea, ichthyosis, vitiligo, urticaria and seborrhoeic dermatitis, and dysbiosis.

13. A pharmaceutical formulation comprising an effective pharmaceutical amount of the composition according to any of the claims 1 to 12, and at least one pharmaceutically acceptable excipient for use in the treatment of skin disorders.

14. The pharmaceutical formulation for use, according to the preceding claim, wherein the at least one pharmaceutical acceptable excipient is suitable for topical or oral administration.

15. Cosmetic use of the postbiotic composition according to any of the preceding claims 1 to 12, or the pharmaceutical formulation according to any of the preceding claims 13 to 14 by improving the appearance of skin or at least one sign of skin aging

Figure 1.a

Figure 1.b

IL-8 secreción THP-1

Figure 2.a

TNF-α secreción THP-1

Figure 2.b

Figure 3

Figure 4.a

Figure 4.b

Figure 5

Figure 6

**Dose range finding**

**Postbiotic Composition**

Figure 7. a

**Dose range finding**

**SDS**

Figure 7. b

Phototoxicity
Postbiotic Composition

Figure 8. a

Phototoxicity
CPZ

Figure 8. b

Europäisches Patentamt

European Patent Office

Office européen des brevets

# EUROPEAN SEARCH REPORT

**Application Number**

EP 23 38 2797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 113 925 884 A (CHAMBIO CO LTD) 14 January 2022 (2022-01-14) * abstract * * claims 1, 4 * * paragraphs [0004], [0006] * * example 3 * | 1-15 | INV. A61K35/741 A61K35/742 A61K35/744 A61K35/745 A61K35/747 A61P17/00 |
| Y | GUENICHE AUDREY ET AL: "Advances in Microbiome-Derived Solutions and Methodologies Are Founding a New Era in Skin Health and Care", PATHOGENS, vol. 11, no. 2, 20 January 2022 (2022-01-20), page 121, XP055943801, DOI: 10.3390/pathogens11020121 * page 11, paragraph 5 - paragraph 7 * * page 12, paragraphs 7, 8, 10 * | 1-15 | A61P17/02 A61P17/04 A61P17/06 A61P17/08 A61P17/10 A23L33/135 A23L33/14 A61K8/9728 A61K8/99 |
| Y | WO 2013/153358 A1 (UNIV MANCHESTER [GB]) 17 October 2013 (2013-10-17) * claims 1, 2, 5, 11, 13 * * page 42, line 6 - line 19 * | 1-15 | |
| Y | CN 107 184 958 A (TAICANG YOUHUO BIOTECHNOLOGY CO LTD) 22 September 2017 (2017-09-22) * claims 1, 3 * * Examples * | 1-15 | **TECHNICAL FIELDS SEARCHED (IPC)** A61K A61P A23L |
| Y | US 11 517 597 B2 (CHAMBIO CO LTD [TW]) 6 December 2022 (2022-12-06) * example 1 * * column 14, paragraph 1 * * claim 1 * | 1-15 | |

The present search report has been drawn up for all claims

| Place of search | Date of completion of the search | Examiner |
|---|---|---|
| Munich | 10 November 2023 | Böhmerova, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 38 2797

This annex lists the patent family members relating to the patent documents cited in  the above-mentioned European search report.
The members are as contained in the European Patent Office EDP file on
The European Patent Office is in no way liable for these particulars which are merely given for the purpose of information.

10-11-2023

| Patent document cited in search report | | | Publication date | Patent family member(s) | | | Publication date |
|---|---|---|---|---|---|---|---|
| CN 113925884 | A | | 14-01-2022 | NONE | | | |
| WO 2013153358 | A1 | | 17-10-2013 | AU | 2013246701 | A1 | 06-11-2014 |
| | | | | BR | 112014025469 | A2 | 01-06-2021 |
| | | | | CA | 2908812 | A1 | 17-10-2013 |
| | | | | CN | 104582712 | A | 29-04-2015 |
| | | | | EP | 2836223 | A1 | 18-02-2015 |
| | | | | EP | 3888628 | A1 | 06-10-2021 |
| | | | | ES | 2883926 | T3 | 09-12-2021 |
| | | | | HK | 1201208 | A1 | 28-08-2015 |
| | | | | JP | 6505594 | B2 | 24-04-2019 |
| | | | | JP | 6944399 | B2 | 06-10-2021 |
| | | | | JP | 2015514127 | A | 18-05-2015 |
| | | | | JP | 2018115168 | A | 26-07-2018 |
| | | | | MX | 362725 | B | 05-02-2019 |
| | | | | RU | 2014145534 | A | 10-06-2016 |
| | | | | US | 2015079040 | A1 | 19-03-2015 |
| | | | | US | 2023088050 | A1 | 23-03-2023 |
| | | | | WO | 2013153358 | A1 | 17-10-2013 |
| CN 107184958 | A | | 22-09-2017 | NONE | | | |
| US 11517597 | B2 | | 06-12-2022 | NONE | | | |

EPO FORM P0459

For more details about this annex : see Official Journal of the European Patent Office, No. 12/82

## EP 4 501 339 A1

**Patent documents cited in the description**

- WO 2022185238 A1
- ES P201930242
- ES P201930280

**Non-patent literature cited in the description**

- *CHEMICAL ABSTRACTS*, 151-21-3
- *CHEMICAL ABSTRACTS*, 298-93-1